(19) Europäisches Patentamt
European Patent Office
Office européen des brevets

(11) EP 2 044 957 A1

(12) **EUROPEAN PATENT APPLICATION**
published in accordance with Art. 153(4) EPC

(43) Date of publication:
**08.04.2009 Bulletin 2009/15**

(21) Application number: **07745569.9**

(22) Date of filing: **26.06.2007**

(51) Int Cl.:
***A61K 45/00*** (2006.01) ***C07D 401/04*** (2006.01)
***A61K 31/4439*** (2006.01) ***A61K 31/444*** (2006.01)
***A61K 31/506*** (2006.01) ***A61P 1/00*** (2006.01)
***A61P 1/04*** (2006.01) ***A61P 11/00*** (2006.01)
***A61P 11/02*** (2006.01) ***A61P 11/06*** (2006.01)
***A61P 17/00*** (2006.01) ***A61P 19/02*** (2006.01)
***A61P 29/00*** (2006.01) ***A61P 37/08*** (2006.01)
***A61P 43/00*** (2006.01) ***C07D 401/14*** (2006.01)
***C07D 413/04*** (2006.01)

(86) International application number:
**PCT/JP2007/063206**

(87) International publication number:
**WO 2008/001929 (03.01.2008 Gazette 2008/01)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **28.06.2006 JP 2006177953**

(71) Applicant: **ASKA Pharmaceutical Co., Ltd.**
**Minato-ku, Tokyo 108-8532 (JP)**

(72) Inventors:
- **HASUMI, Koichi**
  **Kanagawa 213-8522 (JP)**
- **OHTA, Shuji**
  **Kanagawa 213-8522 (JP)**
- **SAITO, Takahisa**
  **Kanagawa 213-8522 (JP)**
- **SATO, Shuichiro**
  **Kanagawa 213-8522 (JP)**
- **KATO, Jun-ya**
  **Kanagawa 213-8522 (JP)**
- **SATO, Jun**
  **Kanagawa 213-8522 (JP)**
- **SUZUKI, Hiroyuki**
  **Kanagawa 213-8522 (JP)**
- **ASANO, Hajime**
  **Kanagawa 213-8522 (JP)**
- **OKADA, Mami**
  **Kanagawa 213-8522 (JP)**
- **MATSUMOTO, Yasuhiro**
  **Kanagawa 213-8522 (JP)**
- **SHIROTA, Kazuhiko**
  **Kanagawa 213-8522 (JP)**

(74) Representative: **Albrecht, Thomas**
**Kraus & Weisert**
**Patent- und Rechtsanwälte**
**Thomas-Wimmer-Ring 15**
**80539 München (DE)**

# (54) TREATMENT AGENT FOR INFLAMMATORY BOWEL DISEASE

(57) The invention discloses treating agents of inflammatory bowel disease, which contain p38MAPkinase inhibitor having properties of antedrug as the active ingredient.

EP 2 044 957 A1

## Description

### Technical Field

This invention relates to treating agent of inflammatory bowel disease, the agent being characterized by containing p38MAPkinase inhibitor having properties of antedrug as the active ingredient.

### Background Art

Inflammation is a reaction of living organism when a certain injurious factor is inflicted on biotissue, which is understood to be a reaction to a chemical mediator for the inflammation which is a result of damage on the tissue, rather than a direct reaction to the tissue damage. As such chemical mediators, for example, tumor necrosis factor-α (TNF-α), interleukin (IL), cyclooxygenase (COX), prostaglandins, thromboxane, leukotriene and the like are known.

On the other hand, the role of p38MAPkinase in inflammatory reaction was clarified in the recent years. Cytokines or proteins such as TNF-α, IL-1, IL-6, IL-8, COX-11 and the like are synthesized when transcription factors such as NF-κ (kappa) B, AP-1, CREB or the like bind to the sequences common among DNAs encoding these cytokines or proteins. P38MAPkinase has the action to activate these transcription factors. When the transcription factors are activated, transcription of mRNA is promoted, and whereby synthesis of cytokines or proteins such as TNF-α is also promoted. Thus p38MAPkinase is positioned upstream in the inflammation reaction route and promotes synthesis of cytokines or proteins such as TNF-α and the like, and hence inhibition of p38MAPkinase is expected to be effective for treatment or prophylaxis of inflammatory diseases, for example, rheumatoid arthritis, osteoarthritis, Crohn's disease, cerative colitis (hereinafter Crohn's disease and ulcerative colitis may be collectively referred to as "IBD" (Inflammatory Bowel Disease)), bronchitis, bronchial asthma, allergic rhinitis, atopic dermatitis and the like.

In the past, as the compounds having p38MAPkinase-inhibiting action, for example, imidazole derivatives (cf. Bioorganic & Medicinal Chemistery, Vol. 5, No. 1, 49 - 64 (1997) and PCT International Publication WO93/14081 Pamphlet), pyrazole derivatives (cf. PCT International Publications WO98/52940 Pamphlet and WO00/39116 Pamphlet) and isoxazole derivatives (cf. JP 2000-86657A and PCT International Publications WO96/25405, WO2004/17968 and WO2004/22555 Pamphlets), thiazole derivatives (cf. PCT International Publication WO00/64894 Pamphlet), triazolopyridine derivatives (cf. PCT International Publication WO2004/72072 Pamphlet), pyridopyrimidine derivatives (cf. PCT International Publication WO2004/14907 Pamphlet), naphthyridine derivatives (cf. PCT International Publication WO2004/73628 Pamphlet), 6-membered ring condensed pyrazole derivatives (cf. PCT International Publications WO2005/73189 and WO2005/85249 Pamphlets), dicyclic hetero-aromatic compounds (cf. PCT International Publication WO2004/00846 Pamphlet) and the like are known.

Among these compounds, some were given, or are being given, clinical investigations. For example, application of a compound which is an imidazole derivative referred to as "Development No. SB-203580" to heumatoid arthritis or asthma has been examined. Also a compound which is a thiazole derivative referred to as "Development No. TAK-715" is currently under clinical trials, making rheumatoid arthritis the indication. However, there is no p38MAPkinase inhibitor marketed as a medicine to date.

Because of its acting mechanism, development of p38MAPkinase inhibitor has been advanced with heumatoid arthritis as its main indication. For a systemic disease like rheumatoid arthritis, it is generally necessary to have a medicine act systemically for a fixed duration of time. Whereas, heretofore investigated p38MAPkinase inhibitors gave rise to such problems as CNS penetration, hepatotoxicity or nephrotoxicity, which rendered it difficult for them to exhibit the medicinal effect by maintaining their constant circulating level, and their development as treating agent of systemic disease had to be abandoned.

Only recently it was reported that a certain kind of triazine derivatives possessed potent p38MAPkinase-inhibiting action and because of their high speed metabolism, were expected to show reduced side effects, and to be prospective antirheumatic medicine (cf. J. Med. Chem., Vol. 47, 6283-6291 (2004)).

On the other hand, steroid anti-inflammatory medicines are very useful for treating inflammatory disease because of their potent anti-inflammatory action, but when they are internally used, their side-effects such as Cushing's syndrome, cataract, glaucoma, suppression of the pituitary-adrenal axis, induction of diabetes, induction of hypertension, deterioration or induction of infectious disease raise problems. Whereas, some of the compounds such as prednisolone valerate acetate, budesonide and the like show little systemic side-effect, as they migrate into blood and quickly metabolize after expressing local anti-inflammatory action. Such medicines which act locally and then migrate into blood, quickly metabolize and disappear are called "antedrug".

Heretofore known medicines as antedrug, however, are mainly externally used steroid compounds, and no antedrug is known as to p38MAPkinase inhibitor. Nor there has been any example of using an antedrug for treating IBD.

## Disclosure of the Invention

The object of the present invention is to offer treating agents of inflammatory bowel disease, which contain p38MAPkinase inhibitor as the active ingredient and show reduced side-effects accompanying systemic distribution of the medicines.

We discovered the existence of compound groups among p38MAPkinase inhibitors, which quickly metabolize after migration into blood and disappear, i.e., have the properties of antedrug, and carried out various investigations with an expectation that the compounds would show little side-effect and be useful for treating inflammatory bowel disease, and now come to complete the present invention.

Thus, according to the present invention, treating agents of inflammatory bowel disease are offered, which are characterized by containing p38MAPkinase inhibitor having properties of antedrug as the active ingredient.

In the present invention, "p38MAPkinase inhibitor having properties of antedrug" signifies a compound having p38MAPkinase-inhibiting action, which migrates into blood after acting locally, wherein a predominant part of the migrated amount, for example, more than a half, quickly metabolizes and is inactivated into a compound exhibiting no p38MAPkinase-inhibiting action. Here the term, "quickly" signifies within an hour, preferably within 30 minutes, inter alia, within 20 minutes.

The effect of the present invention is achieved by the use of p38MAPkinase inhibitor having the properties of antedrug for the treating agent of the present invention. Therefore, the kind of p38MAPkinase inhibitor to be used in the treating agent of the invention is not particularly limited, so long as it has the properties of an antedrug.

Many compounds having p38MAPkinase-inhibiting action have heretofore been reported, but not all of them possess the properties of an antedrug. Screening of p38MAPkinase inhibitors having properties of an antedrug can be easily done by, for example, measuring test of metabolic rate of each compound, as described later.

Thus, specific examples of compounds which can be used as the p38MAPkinase inhibitors having properties of an antedrug in the treating agent of the present invention include:

(A) compounds represented by a formula (I)

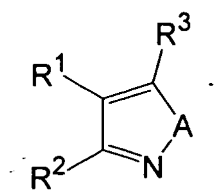

( I )

in the formula,

$R^1$ stands for a substituted or unsubstituted 6-membered aromatic heterocyclic group,
$R^2$ stands for a substituted or unsubstituted aromatic carbocyclic group or aromatic heterocyclic group,
$R^3$ stands for a substituted or unsubstituted carbocyclic group or heterocyclic group, having a straight chain linker, the chain being constituted of 1 - 5 atoms selected from carbon, oxygen and nitrogen, and

A stands for carbon atom, oxygen atom, sulfur atom or nitrogen atom, or salts thereof;

(B) compounds represented by a formula (II)

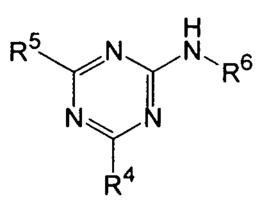

( II )

in the formula,

$R^4$ stands for a heterocyclic group linked directly or through a linker to triazine ring,
$R^5$ stands for a substituted or unsubstituted amino group, and
$R^6$ stands for a substituted or unsubstituted phenyl group, or salts thereof; and

(C) compounds represented by a formula (III)

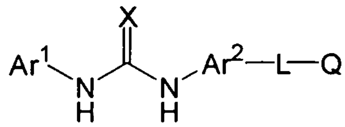

( III )

in the formula,

$Ar^1$ stands for a substituted or unsubstituted aromatic carbocyclic group or aromatic heterocyclic group,
$Ar^2$ stands for a substituted or unsubstituted aromatic carbocycle or heterocycle,
X stands for O or S,
L stands for a linker whose chain is constituted of 1 - 3 atoms selected from carbon, oxygen and sulfur, and
Q stands for a substituted or unsubstituted heterocyclic group,

or salts thereof.

It should be understood that compounds which do not have the properties of an antedrug are excluded from those useful in the present invention, even when they are encompassed by the general formulae (I) - (III).

In the present specification, "inflammatory bowel disease" collectively refers to Crohn's disease and ulcerative colitis which are and generally called by an abbreviated name of "IBD". The present invention has high significance in that it provides useful treating agents of Crohn's disease and ulcerative colitis for which useful therapeutic agent does not exist at the present time.

In the present specification, the term, "lower" signifies that the groups affixed with this prefix each has a carbon number not more than 6, preferably not more than 4.

"Lower alkyl" in this specification may be linear or branched, examples of which include methyl, ethyl, n-propyl, isopropyl, n-butyl, isobutyl, sec-butyl, tert-butyl, n-pentyl and n-hexyl. "Lower alkoxy" are the oxy (O) groups to which the lower alkyl groups are bound, their examples including methoxy, ethoxy, n-propoxy, isopropoxy, n-butoxy, isobutyloxy, sec-butyloxy, tert-butyloxy, n-pentyloxy and n-hexyloxy.

"Lower alkylamino" in this specification signifies an amino group ($-NH_2$) whose one of hydrogen atoms is substituted with above-named lower alkyl group, and "di-lower alkylamino" signifies an amino group whose two hydrogen atoms are substituted with either the same or mutually different lower alkyl groups named in the above. "Lower aralkylamino" signifies a group formed by substituting the lower alkyl moiety in aforesaid lower alkylamino group with aryl group, and "acylamino" signifies an amino group acylated with a lower alkanoyl group such as formyl, acetyl, propionyl, butyryl or the like or an aroyl group such as benzoyl.

Furthermore, "lower alkylthio" and "lower alkylsulfinyl" in this specification respectively signify thio (S) and sulfinyl (SO) groups to which aforesaid lower alkyl groups are bound.

In this specification, "halogen atom" includes fluorine, chlorine, bromine and iodine.

"Substituted or unsubstituted 6-membered aromatic heterocyclic group" in the definition of $R^1$ in the formula (I) include pyridyl, pyridazinyl, pyrimidinyl, pyrazinyl and triazinyl which may be substituted with 1 - 3 substituents selected from halogen, lower alkyl, lower alkoxy, amino, lower alkylamino, di-lower alkylamino, lower aralkylamino, acylamino, lower alkylthio, lower alkylsulfinyl, carboxyl, lower alkoxycarbonyl, aminocarbonyl, lower alkylaminocarbonyl, cyano and nitro. In particular, pyridyl or pyrimidinyl which may be substituted with one substituent selected from the above enumerated substituents are preferred.

"Substituted or unsubstituted aromatic carbocyclic group or aromatic heterocyclic group" in the definition of $R^2$ in the formula (I) include phenyl, naphthyl, pyridyl, pyrimidinyl, furyl, thienyl, pyrrolyl, imidazolyl, pyrazolyl, oxazolyl, isoxazolyl and thiazolyl, which may be substituted with 1 - 3 substituents similar to those above-enumerated. In particular, phenyl or pyridyl which are optionally substituted with 1 - 3 of such substituents are preferred.

As the "straight chain linker, the chain being constituted of 1 - 5 atoms selected from carbon, oxygen and nitrogen" in the definition of $R^3$ in the formula (I), for example, $CH_2$-, -CO-, -O-, -NH-, $-CH_2CH_2-$, $-COCH_2-$, $-CH_2O-$, $-OCH_2-$, -CONH-, $-NHCH_2-$, -NHCO-, $-CH_2CH_2CH_2-$, $-CH_2CH_2CO-$, $-CH_2CH_2O-$, $-OCH_2CH_2-$, $-CH_2OCH_2-$, $-CH_2CH_2NH-$, $-NHCH_2CH_2-$, $-NHCOCH_2-$, $-CONHCH_2-$, $-CH_2CH_2CH_2CH_2-$, $-COCH_2CH_2CH_2-$, $-CH_2CH_2CONH-$,

$-NHCOCH_2CH_2-$, $-CH_2CH_2CH_2CH_2CH_2-$, $-COCH_2CH_2CH_2CH_2-$ and $-NHCOCH_2CH_2CH_2-$ can be named. In particular, the linkers whose chain is constituted of 2 - 4 atoms are preferred. Also the "substituted or unsubstituted carbocyclic or heterocyclic group" in the definition of $R^3$ in the formula (I) are carbocyclic or heterocyclic group which may be substituted with 1 - 3 groups selected from the substituents exemplified as to the "substituted or unsubstituted 6-membered aromatic heterocyclic group" in the above definition of $R^1$. Examples of the carbocyclic group include phenyl, naphthyl, cyclopentyl, cyclohexyl and cycloheptyl. Also examples of the heterocyclic group include furyl, pyridyl, pyrrolidinyl, piperidinyl and azepinyl. Of these, phenyl which is substituted with 1 or 2 groups selected from those substituents exemplified as to the "substituted or unsubstituted 6-membered aromatic heterocyclic group" in the definition of $R^1$ is preferred.

Where A stands for carbon or nitrogen atom in the formula (I), the atom can have one substituent, and as examples of the substituent, lower alkyl or phenyl can be named. In the present invention, A in the formula (I) preferably stands for oxygen, sulfur or nitrogen atom.

The "linker" in the definition of $R^4$ in the formula (II) can be similar to that as explained as to the "straight chain linker, the chain being constituted of 1 - 5 atoms selected from carbon, oxygen and nitrogen" in the definition of $R^3$. Here the heterocyclic group in the definition of $R^4$ preferably is linked to a triazine ring, either directly or through a linker whose chain is constituted of single atom. Examples of the "heterocyclic group" in the definition of $R^4$ in the formula (II) include furyl, pyrrolyl, thienyl, imidazolyl, pyrazolyl, oxazolyl, isoxazolyl, thiazolyl, isothiazolyl, pyridyl, pyrazinyl, pyrimidinyl, pyridazinyl, indolyl, benzimidazolyl, benzoxazolyl, benzthiazolyl, quinolyl, isoquinolyl, quinazolyl, pyrrolidinyl, piperidinyl, piperazinyl, azepinyl and diazepinyl.

"Substituted or unsubstituted amino" in the definition of $R^5$ in the formula (II) specifically includes amino, lower alkylamino, di-lower alkylamino and cycloalkylamino, examples of cycloalkyl in the cycloalkylamino including cyclopropyl, cyclobutyl,-cyclopentyl, cyclohexyl, cycloheptyl and cyclooctyl.

As the substituents on the phenyl group in the "substituted or unsubstituted phenyl" in the definition of $R^6$ in the formula (II), those similar to the substituents exemplified as to the "substituted or unsubstituted 6-membered aromatic heterocyclic group" in the definition of $R^1$ in the formula (I) can be named, and the phenyl can be substituted with 1 - 3 groups selected from these substituents.

"Substituted or unsubstituted aromatic carbocyclic group or aromatic heterocyclic group" in the definition of $Ar^1$ in the formula (III) can be similar to those exemplified as to the "substituted or unsubstituted aromatic carbocyclic group or aromatic heterocyclic group" in the definition of $R^2$ in the formula (I). In particular, phenyl, naphthyl or pyridyl which are substituted with 1 - 3 groups selected from the substituents as exemplified as to the "substituted or unsubstituted 6-membered aromatic heterocyclic ring" are preferred.

"Substituted or unsubstituted aromatic carbocycle or heterocycle" in the definition of $Ar^2$ in the formula (III) include aromatic carbocycle or heterocycle which are optionally substituted with 1 or 2 groups selected from the substituents as exemplified as to the "substituted or unsubstituted 6-membered aromatic heterocyclic group" in the definition of $R^1$ in the formula (I). As the aromatic carbocycle, for example, phenylene and naphthylene can be named, and as the heterocycle, those groups as exemplified as to the "heterocyclic group" in the definition of $R^4$ in the formula (II) can be named.

Examples of the "linker whose chain is constituted of 1 - 3 atoms selected from carbon, oxygen and sulfur" in the definition of L in the formula (III) include $CH_2-$, -O-, -S-, $-CH_2CH_2-$, $-CH_2O-$, $-CH_2S-$, $-OCH_2-$, $-SCH_2-$, $-CH_2CH_2CH_2-$, $-CH_2CH_2O-$, $-CH_2CH_2S-$, $-OCH_2CH_2-$, $-SCH_2CH_2-$, $-CH_2OCH_2-$ and $-CH_2SCH_2-$. Of these, the linkers whose chain is constituted of 1 or 2 atoms selected from carbon, oxygen and sulfur are preferred.

"Substituted or unsubstituted heterocyclic group" in the definition of Q in the formula (III) include heterocyclic groups optionally substituted with 1 - 3 groups selected from the - substituents as exemplified as to the "substituted or unsubstituted 6-membered aromatic heterocyclic group" in the definition of $R^1$ in the formula (I). As examples of the heterocyclic group, those exemplified as to the "heterocyclic group" in the definition of $R^4$ in the formula (II) can be named.

As specific compounds useful for the treating agent of the present invention, the following can be named:

as compounds of the formula (I),
5-[(2-chlorophenyl)acetylaminol-3-(4-fluorophenyl)-4-(4-pyrimidinyl)isoxazole,
5-[(2-chloro-6-fluorophenyl)acetylamino]-3-(4-fluorophenyl)-4-(4-pyrimidinyl)isoxazole,
3-(4-chlorophenyl)-5-[(2-chlorophenyl)acetylamino]-4-(4-pyrimidinyl)isoxazole,
5-[(2-chlorophenyl)acetylamino]-3-(2,4-difluorophenyl)-4-(4-pyrimidinyl)isoxazole,
3-(2,4-difluorophenyl)-5-[(3-methylphenyl)acetylamino]-4-(4-pyrimidinyl)isoxazole,
5-[(2-chlorophenyl)acetylamino]-3-(2-fluoro-4-methoxyphenyl)-4-(4-pyrimidinyl)isoxazole,
5-[(2-chlorophenyl)acetylamino]-3-(2,3-methylenedioxyphenyl)-4-(4-pyrimidinyl)isoxazole,
5-[(2-chlorophenyl)acetylamino]-3-(3-methylphenyl)-4-(4-pyrimidinyl)isoxazole,
5-[(2-bromophenyl)acetylamino]-3-(3-methylphenyl)-4-(4-pyrimidinyl)isoxazole,
3-(3-methylphenyl)-5-[(2-methylphenyl)acetylamino]-4-(4-pyrimidinyl)isoxazole,
3-(3-methylphenyl)-5-[(3-methylphenyl)acetylamino]-4-(4-pyrimidinyl)isoxazole,

3-(2-fluoro-5-methylphenyl)-5-(phenylacetylamino)-4-(4-pyrimidinyl)isoxazole,
5-[(3-methoxyphenyl)acetylamino]-3-(3-methyl-4-fluorophenyl)-4-(4-pyrimidinyl)isoxazole,
3-(3-methyl-4-fluorophenyl)-5-[(2-methylphenyl)acetylamino]-4-(4-pyrimidinyl)isoxazole,
3-(3-methylphenyl)-5-(3-phenylpropionylamino)-4-(4-pyridyl)isoxazole,
3-(3-methylphenyl)-5-[(2-methylphenyl)propionylamino]-4-(4-pyridyl)isoxazole,
5-[(3-chlorophenyl)propionylamino]-3-(2-fluoro-5-methylphenyl)-4-(4-pyridyl)isoxazole,
3-(4-fluoro-3-methylphenyl)-5-phenylacetylamino-4-(4-pyridyl)isoxazole,
5-[(2-chlorophenyl)acetylamino]-3-(4-fluoro-3-methylphenyl)-4-(4-pyridyl)isoxazole,
3-(4-fluoro-3-methylphenyl)-5-(3-phenylpropionylamino)-4-(4-pyridyl)isoxazole,
4-(4-fluorophenyl)-2-(4-hydroxy-3,5-diphenyl)-5-(4-pyridyl)-imidazole,
4-(4-fluorophenyl)-2-(4-methanesulfonylphenyl)-5-(4-pyridyl)-imidazole,
3-(4-fluorophenyl)-5-phenylacetylamino-4-(4-pyridyl)pyrazole,
3-(4-fluorophenyl)-5-(4-methoxyphenylacetylamino)-4-(4-pyridyl)pyrazole,
5-(2-chlorophenylacetylamino)-3-(4-fluorophenyl)-4-(4-pyridyl)pyrazole,
5-(2,5-difluorophenylacetylamino)-3-(4-fluorophenyl)-1-methyl-4-(4-pyridyl)pyrazole,
5-(2-chloro-6-fluorophenylacetylamino)-3-(4-fluorophenyl)-1-methyl-4-(4-pyridyl)pyrazole,
5-(2,6-dichlorophenylacetylamino)-3-(4-fluorophenyl)-1-methyl-4-(4-pyridyl)pyrazole,
5-(2,4-dichlorophenylacetylamino)-3-(4-fluorophenyl)-1-methyl-4-(4-pyridyl)pyrazole,
5-(2,6-difluorophenylacetylamino)-3-(4-fluorophenyl)-1-methyl-4-(4-pyridyl)pyrazole,
5-(3,5-difluorophenylacetylamino)-3-(4-fluorophenyl)-1-methyl-4-(4-pyridyl)pyrazole,
5-(2,3-difluorophenylacetylamino)-3-(4-fluorophenyl)-1-methyl-4-(4-pyridyl)pyrazole,
5-(2,5-dimethylphenylacetylamino)-3-(4-fluorophenyl)-1-methyl-4-(4-pyridyl)pyrazole,
5-(2,4-dimethoxyphenylacetylamino)-3-(4-fluorophenyl)-1-methyl-4-(4-pyridyl)pyrazole,
5-(2-chlorophenylacetylamino)-3-(4-fluorophenyl)-1-methyl-4-(4-pyridyl)pyrazole,
5-(2-bromophenylacetylamino)-3-(4-fluorophenyl)-1-methyl-4-(4-pyridyl)pyrazole,
3-(4-fluorophenyl)-5-(2-fluorophenylacetylamino)-1-methyl-4-(4-pyridyl)pyrazole,
3-(4-fluorophenyl)-1-methyl-5-(2-methylphenylacetylamino)-4-(4-pyridyl)pyrazole,
3-(4-fluorophenyl)-5-(2-methoxyphenylacetylamino)-1-methyl-4-(4-pyridyl)pyrazole,
3-(4-fluorophenyl)-5-(2-nitrophenylacetylamino)-4-(4-pyridyl)-pyrazole,
1-ethyl-3-(4-fluorophenyl)-5-phenylacetylamino-4-(4-pyridyl)-pyrazole,
3-(4-fluorophenyl)-5-phenylacetylamino-1-propyl-4-(4-pyridyl)pyrazole,
3-(4-fluorophenyl)-1-(2-hydroxyethyl)-5-(2-methoxyphenyl-acetylamino)-4-(4-pyridyl)pyrazole,
5-(2-chlorophenylacetylamino)-3-(4-fluorophenyl)-1-(2-hydroxyethyl)-4-(4-pyridyl)pyrazole,
1-methyl-3-(3,4-methylenedioxyphenyl)-5-phenylacetylamino-4-(4-pyridyl)pyrazole,
3-(4-fluorophenyl)-1-methyl-5-phenylacetylamino-4-(4-pyridyl)pyrazole,
3-(4-fluorophenyl)-1-methyl-5-(N-methyl-N-phenylacetyl-amino)-4-(4-pyridyl)pyrazole,
3-(4-fluorophenyl)-5-(N-formyl-N-phenethylamino)-1-methyl-4-(4-pyridyl)pyrazole,
3-(4-fluorophenyl)-5-(1-hydroxy-4-phenylbutyl)-4-(4-pyridyl)-pyrazole,
3-(4-fluorophenyl)-5-[1-hydroxy-3-(3-tolyl)propyl]-4-(4-pyridyl)pyrazole,
3-(4-fluorophenyl)-5-(1-hydroxy-3-phenylbutyl)-4-(4-pyridyl)-pyrazole,
3-(4-fluorophenyl)-5-(1-hydroxy-2-methyl-3-phenylpropyl)-4-(4-pyridyl)pyrazole,
3-(4-fluorophenyl)-5-(1-hydroxy-3-phenylpropyl)-1-methyl-4-(4-pyridyl)pyrazole,
3-(4-fluorophenyl)-4-(4-pyridyl)-5-[3-(2-pyridyl)-1-propenyl]-pyrazole,
3-(4-fluorophenyl)-4-(4-pyridyl)-5-[3-(3-pyridyl)-1-propenyl]-pyrazole,
3-(4-fluorophenyl)-5-[2-methyl-3-(3-pyridyl)-1-propenyl]-4-(4-pyridyl)pyrazole,
3-(4-fluorophenyl)-4-(4-pyridyl)-5-[3-(2-pyridyl)-1-butenyl]-pyrazole,
3-(4-fluorophenyl)-1-methyl-4-(4-pyridyl)-5-[3-(3-pyridyl)-1-propenyl]pyrazole,
3-(4-fluorophenyl)-5-(3-phenylpropyl)-4-(4-pyridyl)pyrazole,
3-(4-fluorophenyl)-4-(4-pyridyl)-5-[3-(3-pyridyl)butyl]pyrazole,
3-(4-fluorophenyl)-4-(4-pyridyl)-5-[3-(2-pyridyl)propyl]-pyrazole,
3-(4-fluorophenyl)-5-[2-methyl-3-(3-pyridyl)propyl]-4-(4-pyridyl)pyrazole,
3-(4-fluorophenyl)-4-(4-pyridyl)-5-[3-(2-pyridyl)butyl]pyrazole,
3-(4-fluorophenyl)-4-(4-pyridyl)-5-[3-(3-pyridyl)propyl]-pyrazole,
5-(3-phenylpropyl)-3-(2-pyridyl)-4-(4-pyridyl)pyrazole,
3-(4-fluorophenyl)-5-[3-(4-nitrophenyl)propyl]-4-(4-pyridyl)-pyrazole,
5-[3-(4-aminophenyl)propyl]-3-(4-fluorophenyl)-4-(4-pyridyl)-pyrazole,
3-(4-fluorophenyl)-5-[3-(1-pyrazolyl)propyl]-4-(4-pyridyl)-pyrazole,
5-[3-(4-aminophenyl)propyl]-3-(4-fluorophenyl)-1-methyl-4-(4-pyridyl)pyrazole,

3-(4-fluorophenyl)-1-methyl-5-(3-phenylpropyl)-4-(4-pyridyl)-pyrazole,
3-(4-fluorophenyl)-1-(2-hydroxyethyl)-5-(3-phenylpropyl)-4-(4-pyridyl)pyrazole,
3-(4-fluorophenyl)-1-(2-dimethylaminoethyl)-5-(3-phenylpropyl)-4-(4-pyridyl)pyrazole,
3-(4-fluorophenyl)-5-(N-methyl-4-methoxybenzylamino-carbonyl)-4-(4-pyridyl)pyrazole,
5-(N-ethylbenzylaminocarbonyl)-3-(4-fluorophenyl)-4-(4-pyridyl)pyrazole,
1-ethyl-5-(N-methylbenzylaminocarbonyl)-3-(4-fluorophenyl)-4-(4-pyridyl)pyrazole,
1-ethyl-5-(N-ethylbenzylaminocarbonyl)-3-(4-fluorophenyl)-4-(4-pyridyl)pyrazole,
3-(4-fluorophenyl)-1-methyl-5-(N-methylbenzylaminocarbonyl)-4-(4-pyridyl)pyrazole,
5-(benzyloxymethyl)-3-(4-fluorophenyl)-4-(4-pyridyl)pyrazole,
5-[1-(benzyloxy)ethyl]-3-(4-fluorophenyl)-4-(4-pyridyl)pyrazole,
3-(4-fluorophenyl)-5-(2-oxo-3-phenylpropyl)-4-(4-pyridyl)-pyrazole, and
3-(4-fluoropheyl)-5-(3-hydroxy-3-phenylpropyl)-4-(4-pyridyl)-pyrazole;

as compounds of the formula (II),

4-methyl-3-[4-[N-methyl-N-(2-phenylpropyl)amino]-6-((S)-pyrrolidin-3-ylamino)-1,3,5-triazin-2-ylamino]benzamide,
4-methyl-3-[4-isopropylamino-6-((S)-pyrrolidin-3-ylamino)-1,3,5-triazin-2-ylamino]benzamide,
4-methyl-3-[4-(N-methyl-N-isopropylamino)-6-((S)-pyrrolidin-3-ylamino)-1,3,5-triazin-2-ylamino]benzamide,
4-methyl-3-[4-(N-methyl-N-neopentylamino)-6-((S)-pyrrolidin-3-ylamino)-1,3,5-triazin-2-ylamino]benzamide,
N-methoxy-4-methyl-3-[4-(N-methyl-N-neopentylamino)-6-(4-methylpiperazin-1-yl)-1,3,5-triazin-2-ylamino]benzamide,
N-methoxy-4-methyl-3-[4-methyl-N-neopentylamino]-6-(piperazin-1-yl)-1,3,5-triazin-2-ylamino]benzamide,
N-methoxy-4-methyl-3-[4-(N-methyl-N-neopentylamino)-6-(4-methyl-1,4-diazepan-1-yl)-1,3,5-triazin-2-ylamino]benzamide,
N-methoxy-4-methyl-3-[4-(N-methyl-N-neopentylamino)-6-(1,4-diazepan-1-yl)-1,3,5-triazin-2-ylamino]benzamide,
N-ethyl-4-methyl-3- [4-(N-methyl-N-neopentylamino)-6-((S)-pyrrolidin-3-ylamino)-1,3,5-triazin-2-ylamino]benzamide,
N-benzyl-4-methyl-3-[4-(N-methyl-N-neopentylamino)-6-((S)-pyrrolidin-3-ylamino)-1,3,5-triazin-2-ylamino]benzamide,
N-methoxy-4-methyl-3- [4-(N-methyl-N-neopentylamino)-6-((S)-pyrrolidin-3-ylamino)-1,3,5-triazin-2-ylamino]benzamide,
N-hydroxy-4-methyl-3-[4-(N-methyl-N-neopentylamino)-6-((S)-pyrrolidin-3-ylamino)-1,3,5-triazin-2-ylamino]benzamide, and
N-isopropyl-4-methyl-3-[4-(N-methyl-N-neopentylamino)-6-((S)-pyrrolidin-3-ylamino)-1,3,5-triazin-2-ylamino]benzamide;

as compounds of the formula (III),

[4-(2-cyanopyridin-4-ylmethyl)-2-fluorophenyl] amino-N-(2-naphthyl)carboxamide,
[3-chloro-4-(2-cyanopyridin-4-ylmethyl)phenyl]amino-N-(2,2-difluorobenzo[d]-1,3-dioxan-5-yl)carboxamide,
[3-chloro-4-(2-cyanopyridin-4-yloxy)phenyl]amino-N-(2,2-difluorobenzo[d]-1,3-dioxan-5-yl)carboxamide,
[3-chloro-4-(2-cyanopyridin-4-ylthio)phenyl]amino-N-(2,2-difluorobenzo[d]-1,3-dioxan-5-yl)carboxamide,
[4-(2-cyanopyridin-4-ylmethyl)-3-methylphenyl]amino-N-(2,2,3,3-tetrafluorobenzo[e]-1,4-dioxan-6-yl)carboxamide,
[4-(2-cyanopyridin-4-yloxy)-3-methylphenyl]amino-N-(2,2,3,3-tetrafluorobenzo[e]-1,4-dioxan-6-yl)carboxamide,
[4-(2-cyanopyridin-4-yloxy)-2-fluorophenyl]amino-N-(2-trifluoromethylpyridin-4-yl)carboxamide,
[4-(2-cyanopyridin-4-yloxy)phenyl]amino-N-(4-tert-butylpyridin-2-yl)carboxamide,
[4-(2-cyanopyridin-4-yloxy)-3-fluorophenyl]amino-N-(4-tert-butylpyridin-2-yl)carboxamide,
[4-(2-cyanopyridin-4-yloxy)phenyl]amino-N-(4-ethylpyridin-2-yl)carboxamide,
[2-chloro-4-(2-cyanopyridin-4-yloxy)phenyl]amino-N-quinolin-6-ylcarboxamide,
[3-chloro-4-(2-cyanopyridin-4-yloxy)phenyl]amino-N-isoquinolin-3-ylcarboxamide,
[4-(2-cyanopyridin-4-yloxy)-2-nitrophenyl]amino-N-benzothiazol-5-ylcarboxamide, and
[4-(3-methylaminocarbonylpyridin-4-yloxy)-2-fluorophenyl]-amino-N-(3-trifluoromethyl-4-chlorophenyl)carboxamide; and the like.

Those compounds which are used for the treating agent of the present invention can optionally be present in the form of salts. As the salts, those with inorganic acid such as hydrochloric acid, hydrobromic acid, sulfuric acid, nitric acid, phosphoric acid and the like; and those with organic acid such as acetic acid, oxalic acid, citric acid, lactic acid, tartaric acid, p-toluenesulfonic acid and the like; alkali metal salts such as sodium salts, potassium salts, lithium salts

and the like; alkaline earth metal salts such as calcium salts, magnesium salts and the like; salts with organic base such as triethylamine, dicyclohexylamine, pyrrolidine, morpholine, pyridine and the like; and ammonium salts can be named. Of these, pharmaceutically acceptable salts are preferred.

The compounds of the formula (I) can be readily produced by the methods described in later appearing Production Examples or in known literature, for example, PCT International Publication WO00/39116 Pamphlet, PCT International Publication WO00/75131 Pamphlet and the like. Also the compounds of the formula (II) can be readily produced, following the methods described in, for example, J. Med. Chem., Vol. 47, 6283-6291 (2004). Furthermore, the compounds of the formula (III) can be readily produced, following the method described in, for example, PCT International Publication WO2004/078747 Pamphlet.

The p38MAPkinase-inhibiting activity, TNF-α production-inhibiting activity and the metabolic rate of the compounds which are to be used for the treating agents of the present invention are demonstrated by the following experiments.

(1) Measurement of inhibitory activity to binding of p38MAPkinase

THP-1 cells were suspended in cell lysis buffer (a liquid mixture of 20 mM tris-hydrochloric acid buffer solution (pH 7.4), 1 mM magnesium chloride, 1 mM phenylmethylsulfonyl fluoride, 1 mM pepstatin A, 1 mM leupeptin and 10 mg/mL aprotinin), and then given an ultrasonic treatment in water. After centrifuging the system at 100,000 x g for an hour, the protein concentration in the resultant supernatant containing the cytosol fraction was measured. The cytosol fraction was diluted with cell lysis buffer to make its protein concentration 1 mg/mL, and divided into small portions which were kept at -80˚C until the time of use.

The binding-inhibiting activity was measured after incubating the cytosol fraction (100 μg protein) of THP-1 cells with the test compound at 15˚C for 30 minutes, adding thereto 1.11 KBq of 4-(4-fluorophenyl)-2-(4-hydroxy-3,5-di-3H-phenyl)-5-(4-pyridyl)-imidazole (925 GBq/mmol, Amersham UK) as radioligand, and further continuing the reaction at 15˚C for 3 hours. Also non-specific binding was measured by adding 20 μM of 4-(4-fluorophenyl)-2-(4-methanesulfonylphenyl)-5-(4-pyridyl)imidazole. For separating free and binding type radioligands, a charcoal solution (1% charcoal and 0.1% dextran T-70) was added, followed by 15 minutes' cooling with ice and centrifugation (3,000 rpm, 10 minutes, 4˚C). The radio activity in the resultant supernatant was measured with liquid scintillation counter. The results of the measurements are shown in the following Table A.

TABLE A

| Compound No. | Structural Formula | $IC_{50}$(nM) |
|---|---|---|
| A-1 | 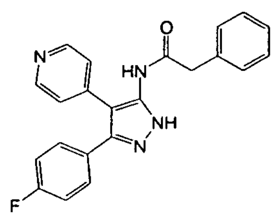 | 0.042 |
| A-2 | 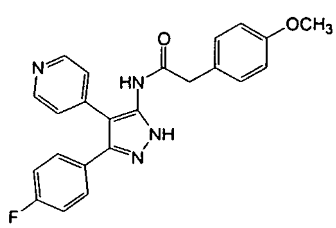 | 0.032 |
| A-3 | 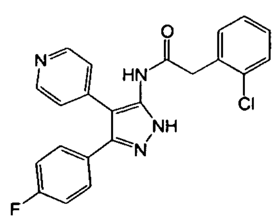 | 0.0023 |

(continued)

| Compound No. | Structural Formula | $IC_{50}$(nM) |
|---|---|---|
| A-4 | 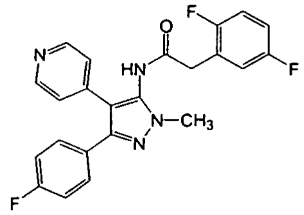 | 0.0012 |
| A-5 | 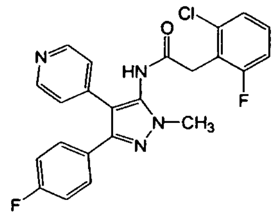 | 0.0061 |
| A-6 | 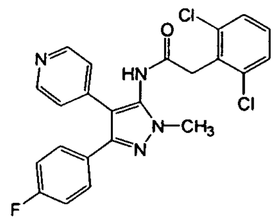 | 0.035 |
| A-7 | 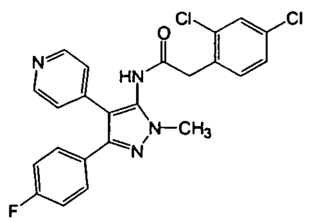 | 0.0017 |
| A-8 | 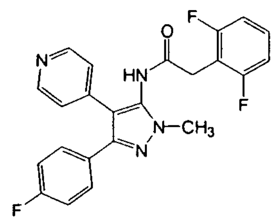 | 0.00043 |
| A-9 | 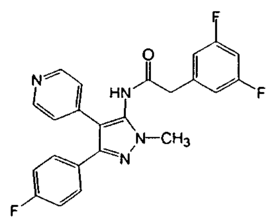 | 0.026 |
| A-10 | 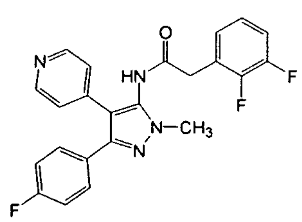 | 0.083 |

(continued)

| Compound No. | Structural Formula | $IC_{50}$(nM) |
|---|---|---|
| A-11 | 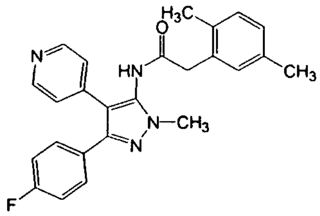 | 0.00021 |
| A-12 | 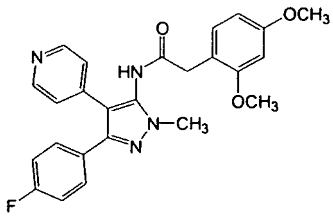 | 0.032 |
| A-13 | 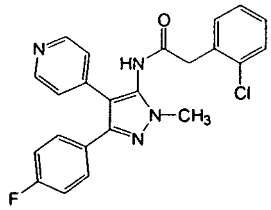 | 0.0000088 |
| A-14 | 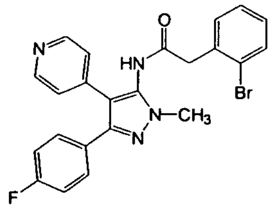 | 0.00084 |
| A-15 | 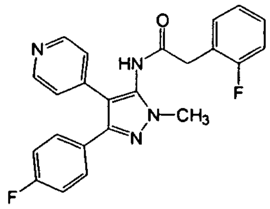 | 0.057 |
| A-16 | 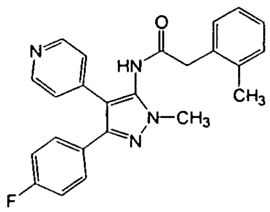 | 0.05 |
| A-17 | 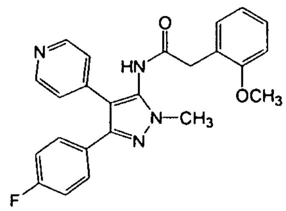 | 0.00016 |

(continued)

| Compound No. | Structural Formula | $IC_{50}$(nM) |
|---|---|---|
| A-18 | 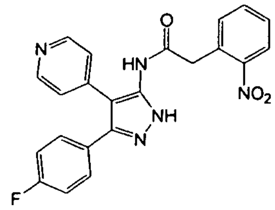 | 0.055 |
| A-19 | 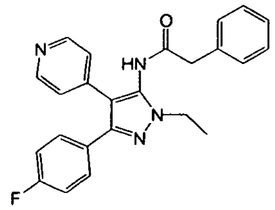 | 0.041 |
| A-20 | 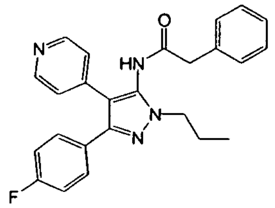 | 0.029 |
| A-21 | 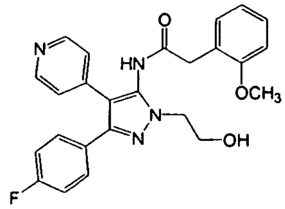 | 0.02 |
| A-22 | 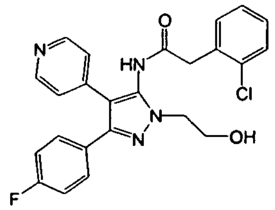 | 0.028 |
| A-23 | 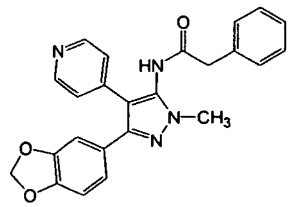 | 0.082 |
| A-24 | 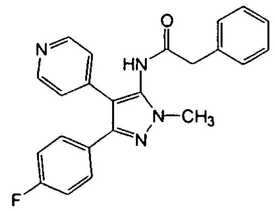 | 0.035 |

(continued)

| Compound No. | Structural Formula | $IC_{50}$(nM) |
|---|---|---|
| A-25 | 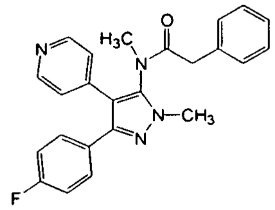 | 0.25 |
| A-26 | 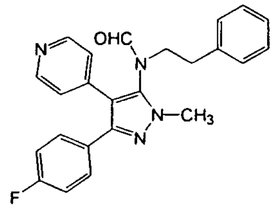 | 0.025 |
| A-27 | 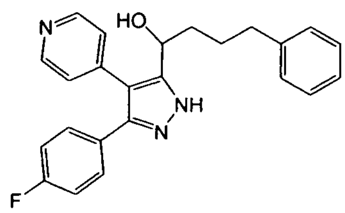 | 0.546 |
| A-28 | 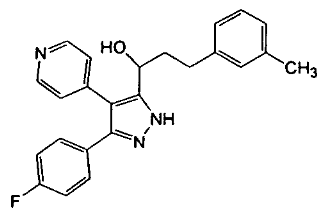 | 0.99 |
| A-29 | 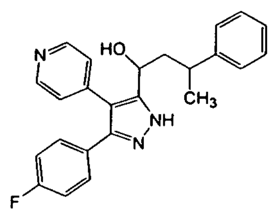 | 0.203 |
| A-30 | 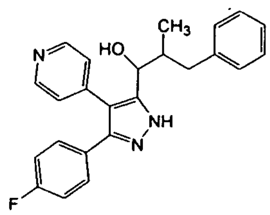 | 0.205 |
| A-31 | 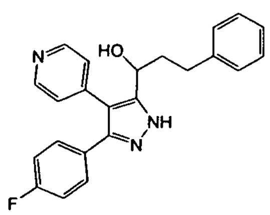 | 0.316 |

(continued)

| Compound No. | Structural Formula | $IC_{50}$(nM) |
|---|---|---|
| A-32 | 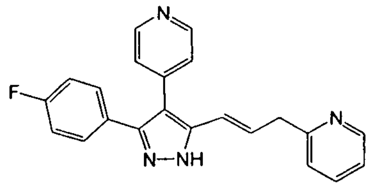 | 0.431 |
| A-33 | 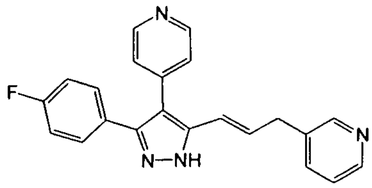 | 0.02 |
| A-34 | 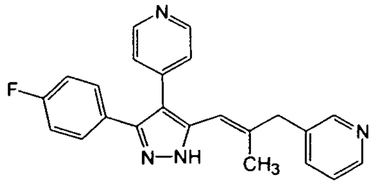 | 0.000786 |
| A-35 | 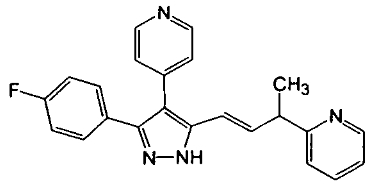 | 0.0579 |
| A-36 | 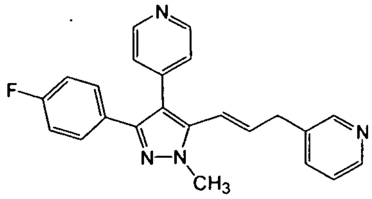 | 0.0956 |
| A-37 | 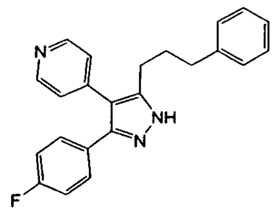 | 0.329 |
| A-38 | 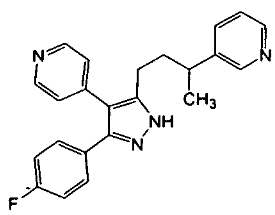 | 0.335 |

(continued)

| Compound No. | Structural Formula | $IC_{50}$(nM) |
|---|---|---|
| A-39 | 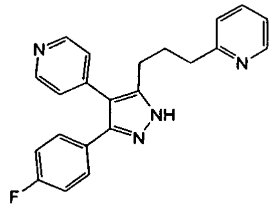 | 0.04 |
| A-40 | 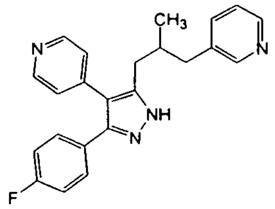 | 0.00851 |
| A-41 | 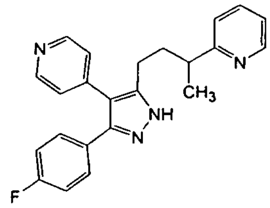 | 0.017 |
| A-42 | 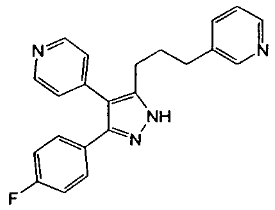 | 0.0000115 |
| A-43 | 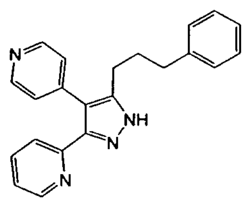 | 0.471 |
| A-44 | 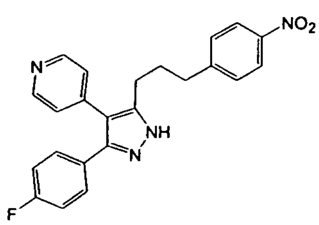 | 0.402 |
| A-45 | 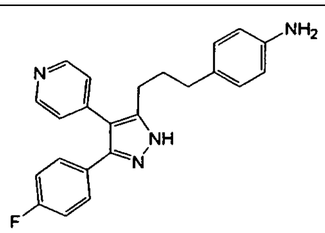 | 0.11 |

(continued)

| Compound No. | Structural Formula | $IC_{50}$(nM) |
|---|---|---|
| A-46 | 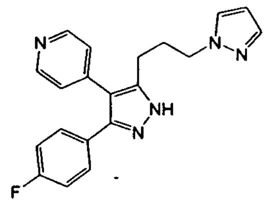 | 0.226 |
| A-47 | 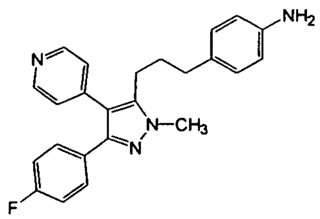 | 0.365 |
| A-48 | 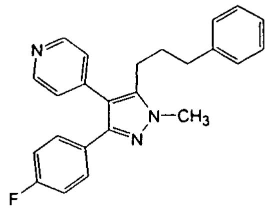 | 0.284 |
| A-49 | 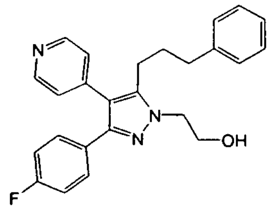 | 0.042 |
| A-50 | 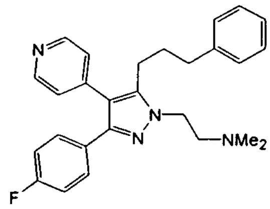 | 0.114 |
| A-51 | 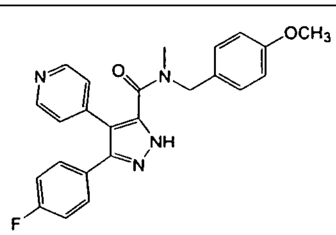 | 0.31 |
| A-52 | 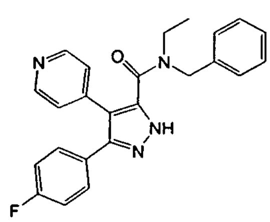 | 0.302 |

(continued)

| Compound No. | Structural Formula | IC$_{50}$(nM) |
|---|---|---|
| A-53 | 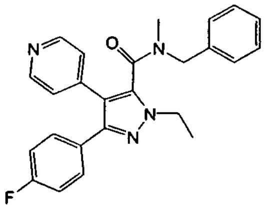 | 0.0484 |
| A-54 | 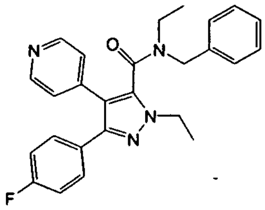 | 0.0279 |
| A-55 | 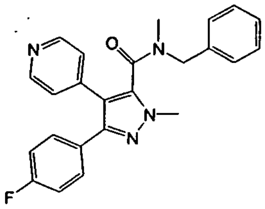 | 0.335 |
| A-56 | 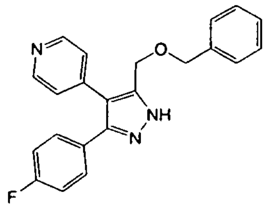 | 0.154 |
| A-57 | 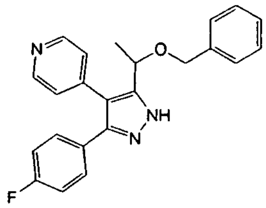 | 0.175 |
| A-58 | 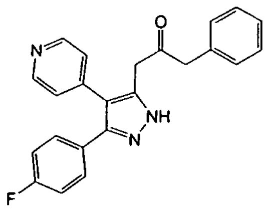 | 0.358 |
| A-59 | 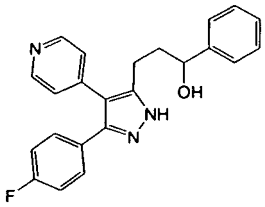 | 0.231 |

(2) Measurement of TNF-α production-inhibiting activity:

THP-1 (purchased from Dainippon Pharmaceuticals Co.) which are human-derived cultured cells, were suspended in RPMI1640 culture medium (10% fetal bovine serum, containing 100 units/mL penicillin) (1 x $10^5$ cells/mL). Onto a 24-well culture plate, 1.6 mL of the THP-1 cell suspension was inoculated, and further 0.2 mL of the test substance as dissolved in RPM11640 culture medium and 0.2 mL of LPS (E. Coli 055: B5-derived, dissolved in RPM11640 culture medium, Difco) at a concentration of 10 μg/mL were added, followed by 2 hours' cultivation under the conditions of 37°C and 5% $CO_2$. The supernatant obtained upon centrifugation (500 x g, 5 min.) was assayed with ELISA (Amersham Bioscience, TNF-α Human, ELISA Biotrak System) to quantify TNF-α. The concentration of each test substance required to inhibit TNF-α production by 50% ($IC_{50}$) was determined as follows. First, TNF-α production inhibition (%) values at various concentrations were determined by the following formula:

$$\left[1-\frac{\text{quantity of TNF-}\alpha\text{ under use of each test substance}}{\text{quantity of TNF-}\alpha\text{ in control experiment}}\right]\times 100 \quad .$$

The TNF-α production inhibition (%) obtained by the above formula and the concentration of the test substance in that occasion were computed on Prism 4 for Windows Ver 4.02 (Graph Pad Software, Inc.) to calculate $IC_{50}$ value.

The results are shown in later-appearing Table B, concurrently with the results of the following (3) measurement of the compounds' extinction rates.

(3) Measurement of the compounds' extinction rates:

To a potassium phosphate buffer solution (50 mmol/L, pH 4.7) containing NADPH production system (formed of 3.3 mmol/L $MgCl_2$, 3.3 mmol/L glucose-6-phosphate, 1.3 mmol/L β-$NADP^+$ and 0.4 unit/mL glucose-6-phosphate dehydrogenase), each test compound was added (in which occasion the final concentration was made 1 μmol/L) and incubated at 37°C for 2 minutes. After the incubation, a suspension of human liver S9 (supernatant fraction obtained by centrifugation of crushed human liver cells at 9000 x g) in potassium phosphate buffer solution was added to a final concentration of 0.5 mg protein/mL. This liquid reaction mixture was incubated at 37°C for 5 minutes, to which 4 volume times thereof of acetonitrile was added, mixed, and cooled with ice. After the cooling with ice, the mixture was centrifuged (2000 x g, 10 min.) and a part of the supernatant was recovered and analyzed with LS/MS/MS to calculate the residual ratio of unchanged substance in the reaction solution. The results are shown in the following Table B concurrently with the results of above (2) measurement of TNF-α production-inhibiting activity.

TABLE B

| Compound No. | Structural Formula | TNF- α Production-inhibiting activity ($IC_{50}$ value: nM) | Extinction Rate (residual ratio of unchanged substance: %) |
|---|---|---|---|
| B-1 | 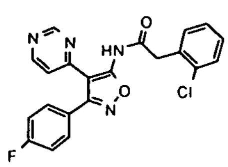 | 36.1 | 5.8 |
| B-2 | 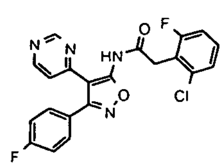 | 67.9 | 16.1 |
| B-3 | 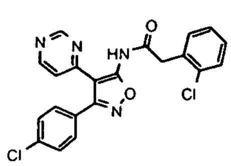 | 48.7 | 53.1 |

(continued)

| Compound No. | Structural Formula | TNF- α Production-inhibiting activity ($IC_{50}$ value: nM) | Extinction Rate (residual ratio of unchanged substance: %) |
|---|---|---|---|
| B-4 | 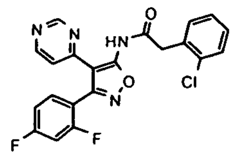 | 32.1 | 20.4 |
| B-5 | 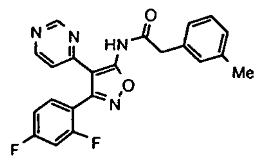 | 139 | 46.6 |
| B-6 | 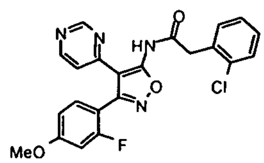 | 28.5 | 24.1 |
| B-7 | 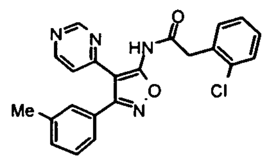 | 36.7 | 21.2 |
| B-8 | 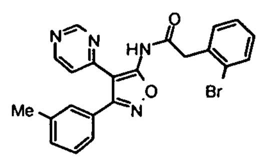 | 49.2 | 43.6 |
| B-9 | 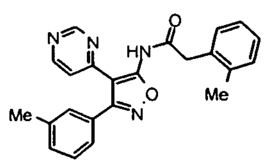 | 13.4 | 59.9 |
| B-10 | 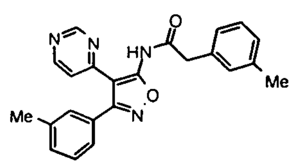 | 152 | 38.5 |
| B-11 | 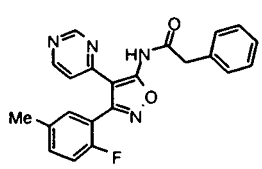 | 90.5 | 39.8 |
| B-12 | 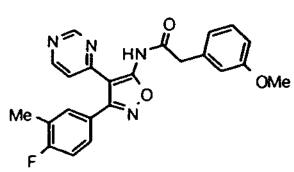 | 52.6 | 33.1 |

(continued)

| Compound No. | Structural Formula | TNF- α Production-inhibiting activity ($IC_{50}$ value: nM) | Extinction Rate (residual ratio of unchanged substance: %) |
|---|---|---|---|
| B-13 | 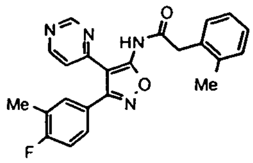 | 13.7 | 52.6 |
| B-14 | 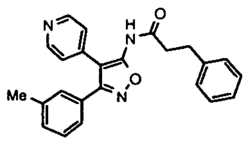 | 65.5 | 17.6 |
| B-15 | 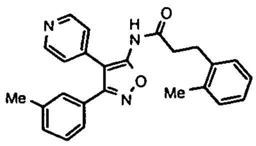 | 52.9 | 23.3 |
| B-16 | 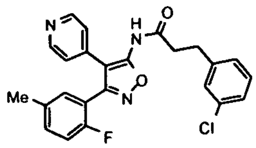 | 58.1 | 50.5 |
| B-17 | 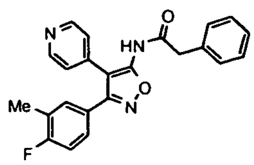 | 78.1 | 58.0 |
| B-18 | 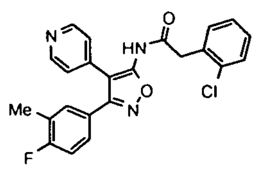 | 85.2 | 59.7 |
| B-19 | 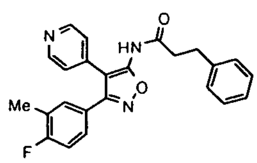 | 66.0 | 20.1 |
| B-20 | 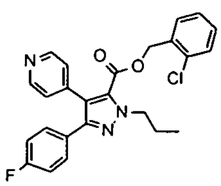 | 83.3 | 77.0 |

(continued)

| Compound No. | Structural Formula | TNF- α Production-inhibiting activity ($IC_{50}$ value: nM) | Extinction Rate (residual ratio of unchanged substance: %) |
|---|---|---|---|
| B-21 | 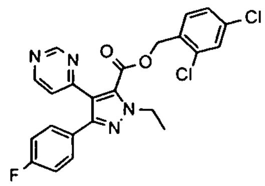 | 61.5 | 56.4 |
| B-22 | 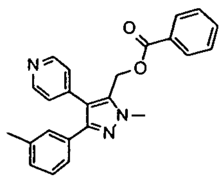 | 45.5 | 25.5 |
| B-23 | 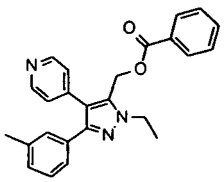 | 26.5 | 28.3 |

The efficacy of the compounds used in the treating agents on pathological models can be demonstrated by the experiments described in the following.

4) DDS-induced colitis model in mice

Colitis models were prepared by having BALB/c female mice drink 5% dextran sodium sulfate (DSS) freely for a week.

Simultaneously with initiation of the drinking, each test substance was orally administered to the test mice twice a day for a week. Also as the reference, salazosulfapyridine was orally administered once a day for a week. One week after initiation of the drinking, the test mice' intestines were extracted, with which the efficacy of test substances was evaluated, using the length of colon + rectum as the index. The results are shown in the later appearing Table C, concurrently with the results of investigation in the following 5) acetic acid-induced colitis model in rats.

5) Acetic acid-induced colitis model in rats

Wistar male rats were fasted for 24 hours, given celiotomy under anesthesia with Nembutal, to expose the caecum and colon. Twenty (20) μL of 20% acetic acid was injected into the submucosa at 5 cm from the caecum toward anus, from the serous membrane side using a microsyringe. Thereafter the injection site was washed with physiological saline, returned into the abdominal cavity, and the operated site was closed, to make colitis models.

Starting on the day of the model preparation, each test substance was intrarectally administered twice a day. Also as the reference, 5-aminosalycilic acid was similarly administered. After 5 days' administration of the test substances, the rats' intestines were extracted and efficacy of each test substance was evaluated, using the area at which the disorder occurred as the index. The results are shown in the following table C, concurrently with the results of the investigation of DDS-induced colitis model in mice.

TABLE C

| Compound No. | Dose confirmed to be effective | |
|---|---|---|
| | mouse DSS | rat acetic acid |
| B-1 | 30 mg | - |
| B-2 | 3 mg | 30 mg |

(continued)

| Compound No. | Dose confirmed to be effective | |
|---|---|---|
| | mouse DSS | rat acetic acid |
| B-4 | 30 mg | - |
| B-7 | 3 mg | 30 mg |
| B-14 | 30 mg | - |
| B-17 | 3 mg, 30 mg | 100 mg |

Thus the treating agents according to the present invention can be orally or parenterally (e.g., intramuscular injection, intravenous injection, intrarectal or percutaneous administration and the like) administered for treating inflammatory bowel disease suffered by human or mammals other than human, as medicines having excellent p38MAPkinase-inhibiting action and little side-effects because they quickly disappear after entering into blood.

The treating agents of the present invention can be formulated into preparation forms according to their utility, with non-toxic excipients, such as solids (e.g., tablet, hard capsule, soft capsule, granule, powder, grain, pill, troche and the like); semi-solids (e.g., supporsitory, ointment and the like) or liquid (e.g., injection, emulsion, suspension, lotion, spray and the like). As the non-toxic excipients useful for such preparations, for example, starch, gelatine, glucose, lactose, fructose, maltose, magnesium carbonate, talc, magnesium stearate, methyl cellulose, carboxymethyl cellulose or salts thereof, gum arabic, polyethylene glycol, alkyl ester of p-hydroxybenzoic acid, syrup, ethanol, propylene glycol, petrolatum, carbowax, glycerine, sodium chloride, sodium sulfite, sodium phosphate, citric acid and the like can be named. The preparations can also contain other therapeutically useful medicines.

Thus, according to the present invention, pharmaceutical compositions for treating inflammatory bowel disease are provided, which contain p38MAPkinase inhibitor having properties of antedrug concurrently with non-toxic excipients.

According to the invention, also a method for treating inflammatory bowel disease is provided, which is characterized by administering to the patients who need the treatment, an effective amount of p38MAPkinase inhibitor having the properties of antedrug.

While the content of the treating agent of the present invention in such preparations or compositions differs according to the preparation form, in general terms it is desirable to be within a concentration range of 0.1 - 50% by weight for solid and semi-solid forms, and within a concentration range of 0.05 - 10% by weight for liquid forms.

The administration dosage of the treating agent of the present invention is variable over a wide range according to the species, age, body weight, administration route, seriousness of symptoms and doctor's diagnosis, of the patients including human and other warm-blooded animals. Whereas, in general terms, it can range 0.02-20 mg/kg, preferably 0.2 - 8 mg/kg, per day. Obviously, dosages less than the lower limit or more than the upper limit of the above-specified range may be administered depending on seriousness of the patient's symptoms, doctor's diagnosis and the like. The dosage can be administered as a single dose or plural divided doses per day.

Examples

Hereinafter the present invention is more specifically explained, referring to Examples and Preparation Examples.

Example 1: Formulation Example of tablets

| | mg/tablet |
|---|---|
| Active ingredient | 5.0 |
| Starch | 10.0 |
| Lactose | 73.0 |
| Carboxymethyl cellulose calcium | 10.0 |
| Talc | 1.0 |
| Magnesium stearate | 1.0 |
| | 100.0 |

The active ingredient is pulverized to a grain size not greater than 70 μm, and to which starch, lactose and carboxymethyl cellulose calcium are added and thoroughly mixed. Ten (10)% starch paste is added to the mixture, mixed by stirring and granulated. After drying, the granules are dressed to around 1000 μm in particle size. Mixing talc and magnesium stearate therewith, the blend is tabletted.

Preparation Example 1

3-(4-Fluorophenyl)-5-(phenylacetylamino)-4-(4-pyrimidinyl)-isoxazole

a: Synthesis of dimethyl-[(E)-2-(4-pyrimidinyl)vinyl]amine

A mixture of 10 g of 4-methylpyrimidine, 38 g of N,N-dimethylformamide dimethylacetal (DMFDMA) and 46.6 g of DMF was stirred in a sealed tube at 140˚C for 24 hours. The reaction solution was cooled and the solvent was distilled off under reduced pressure to provide 15.08 g (yield: 95%) of the title compound as brown crystal.
$^1$H-NMR($CDCl_3$)δ: 8.73(bs, 1H), 8.22(d, J=5.5Hz, 1H), 7.77(d, J=12.9Hz, 1H), 6.72(dd, J=5.5Hz, 12.9Hz, 1H), 5.00(d, J=12.9Hz, 1H), 2.96(s, 6H).

b: Synthesis of 4-pyrimidinylacetonitrile

To 70 mL of an aqueous solution containing 5 g of dimethyl-[(E)-2-(4-pyrimidinyl)vinyl]amine, 9.48 g of hydroxylamine-O-sulfonic acid was added and stirred at 50˚C for 30 minutes. The reaction solution was made basic by addition of saturated aqueous sodium hydrogen carbonate solution under cooling with ice, and extracted with ethyl acetate. The ethyl acetate extract was dried over anhydrous magnesium sulfate and removed of the solvent by distillation under reduced pressure. Thus obtained residue was purified on 30 g silica gel column chromatography (eluent, chloroform: methanol = 30:1) to provide 1.56 g (yield: 39%) of the title compound as pale yellow crystal.
$^1$H-NMR($CDCl_3$)δ: 9.21(d, J=1.2Hz, 1H), 8.80(d, J=5.2Hz, 1H), 7.51(dd, J=1.2Hz, 5.2Hz, 1H), 3.93(s, 2H).

c: Synthesis of 5-amino-3-(4-fluorophenyl)-4-(4-pyrimidinyl)-isoxazole

Sodium methoxide 2.50 g was dissolved in 50 mL of methanol, into which 50 mL of a THF solution containing 5 g of 4-pyrimidinylacetonitrile was dropped, followed by 30 minutes' stirring at room temperature. Then 50 mL of a methanol solution containing 7.29 g of 4-fluorobenzhydroxymoyl chloride was dropped into the solution and stirred at room temperature for 7 hours. After removing the solvent from the reaction solution by distillation under reduced pressure, water was added and the precipitated residue was recovered by filtration, washed with water and dried under reduced pressure. Thus obtained residue was purified on 80 g silica gel column chromatography (eluent, chloroform: methanol = 50:1 - 30:1) and washed with ether to provide 7.86 g (yield: 73%) of the title compound as light gray crystal.
$^1$H-NMR($CDCl_3$)δ: 9.03(d, J=1.4Hz, 1H), 8.32(d, J=5.6Hz, 1H), 7.70-7.05(m, 4H), 6.88(bs, 2H), 6.70(dd, J=1.4Hz, 5.6Hz, 1H).
Mass, m/e: 256($M^+$), 111(base).

d: Synthesis of 3-(4-fluorophenyl)-5-(phenylacetylamino)-4-(4-pyrimidinyl)isoxazole

Imidazole 0.43 g and DBU 1.9 g were dissolved in 40 mL of THF. Under cooling with ice and stirring, 0.97 g of phenylacetyl chloride was dropped into the solution, followed by 20 minutes' stirring at room temperature. Then 40 mL of a THF solution containing 0.8 g of 5-amino-3-(4-fluorophenyl)-4-(4-pyrimidinyl)-isoxazole was dropped into the system and stirred at room temperature for 6 hours. From the reaction solution the solvent was distilled off under reduced pressure and water was added to the residue, which was then extracted with ethyl acetate. The organic layer was dried over anhydrous magnesium sulfate and from which the solvent was distilled off under reduced pressure. Thus obtained residue was purified on 40 g silica gel column chromatography (eluent, chloroform: methanol = 100:1) and washed with ether to provide 0.77 g (yield: 66%) of the title compound in the form of colorless crystal.
$^1$H-NMR($CDCl_3$)δ: 11.39(s, 1H), 8.49(s, 1H), 8.36(d, J=5.6Hz, 1H), 7.50-7.38(m, 7H), 7.20(t, J=8.5Hz, 2H), 6.73(dd, J=1.3Hz, 5.6Hz, 1H), 3.94(s, 2H).
Mass, m/e: 374($M^+$), 240(base).

Preparation Example 2

5-[(2-Chlorophenyl)acetylamino]-3-(4-fluorophenyl)-4-(4-pyrimidinyl)isoxazole (Compound No. B-1)

Preparation Example 1-d was repeated except that phenylacetyl chloride was replaced by 2-chlorophenylacetyl chloride, to synthesize the title compound.
$^1$H-NMR($CDCl_3$)δ:11.45(bs,1H),8.54(s,1H),8.38(d,J=5.7Hz,1H), 7.55-7.38(m,6H),7.20(t,J=8.7Hz,2H), 6.75(dd, J=1.3Hz,5.7Hz,1H),4.06(s,2H)
Mass,m/e:408($M^+$),240(base)

Preparation Example 3

5-[(2-Chloro-6-fluorophenyl)acetylamino]-3-(4-fluorophenyl)-4-(4-pyrimidinyl)isoxazole (Compound No. B-2)

In the manner similar to Preparation Example 2, the title compound was synthesized.
$^1$H-NMR($CDCl_3$)δ:11.55(s,1H),8.64(s,1H),8.40(d,J=5.7Hz,1H), 7.51-7.45(m,2H),7.43-7.34(m,2H),7.26-7.13(m,3H), 6.78(dd,J=1.3Hz,5.7Hz,1H),4.14(s,2H)
Mass,m/e:426($M^+$),240(base)

Preparation Example 4

3-(4-Chlorophenyl)-5-[(2-chlorophenyl)acetylamino]-4-(4-pyrimidinyl)isoxazole (Compound No. B-3)

Preparation Example 1-c was repeated except that 4-fluorobenzhydroxymoyl chloride was replaced by 4-chlorobenzhydroxymoyl chloride to synthesize 5-amino-3-(4-chlorophenyl)-4-(4-pyrimidinyl)isoxazole, from which the title compound was synthesized in the manner similar to Preparation Example 2.
$^1$H-NMR($CDCl_3$)δ:11.44(bs,1H),8.53(s,1H),8.39(d,J=5.6Hz,1H), 7.54-7.39(m,8H),6.76(dd,J=1.5Hz,5.6Hz,1H),4.06(s, 2H)
Mass,m/e:424($M^+$),256(base)

Hereinafter those compounds of Preparation Examples 5 - 14 were synthesized in the manner similar to Preparation Example 4.

Preparation Example 5

5-[(2-Chlorophenyl)acetylamino]-3-(2,4-difluorophenyl)-4-(4-pyrimidinyl)isoxazole (Compound No. B-4)

$^1$H-NMR($CDCl_3$)δ:11.49(bs,1H),8.53(s,1H),8.41(d,J=5.4Hz,1H), 7.54-7.40(m,5H),7.09-7.04(m,1H),6.98(dt, J=2.3Hz,8.5Hz,1H),6. 67(td,J=1.5Hz,5.4Hz,1H),4.07(s,2H)
Mass,m/e:426($M^+$),258(base)

Preparation Example 6

3-(2,4-Difluorophenyl)-5-[(3-methylphenyl)acetylamino]-4-(4-pyrimidinyl)isoxazole (Compound No. B-5)

$^1$H-NMR($CDCl_3$)δ:11.43(s, 1H),8.48(s, 1H),8.40(d,J=5.4Hz, 1H), 7.54-7.49(m,1H),7.40-7.36(m,1H),7.28-7.20 (m,3H), 7.09-7.04(m,1H),7.00-6.95(m,1H),6.76(dt,J=1.5Hz,5.4Hz,1H),3. 89(s,2H),2.40(s,3H)
Mass,m/e:406($M^+$),258(base)

Preparation Example 7

5-[(2-Chlorophenyl)acetylamino]-3-(2-fluoro-4-methoxyphenyl)-4-(4-pyrimidinyl)isoxazole (Compound No. B-6)

$^1$H-NMR($CDCl_3$)δ:11.49(s,1H),8.52(s,1H),8.39(d,J=5.8Hz,1H), 7.54-7.51(m,1H),7.48-7.46(m,1H),7.43-7.39 (m,3H), 6.84(dd,J=2.3Hz,8.5Hz,1H),6.76-6.72(m,2H),4.06(s,2H), 3.87(s,3H)
Mass,m/e:438($M^+$),270(base)

Preparation Example 8

5-[(2-Chlorophenyl)acetylaminol-3-(3-methylphenyl)-4-(4-pyrimidinyl)isoxazole (Compound No. B-7)

$^1$H-NMR($CDCl_3$)δ:8.52(s,1H),8.34(d,J=5.4Hz,1H), 7.54-7.20(m,8H),6.78(dd,J=1.5Hz,5.4Hz,1H),4.07(s,2H), 2.39(s,3H)
Mass,m/e:404($M^+$),236(base)

Preparation Example 9

5-[(2-Bromophenyl)acetylamino]-3-(3-methylphenyl)-4-(4-pyrimidinyl)isoxazole (Compound No. B-8)

$^1$H-NMR($CDCl_3$)δ:11.45(bs,1H),8.51(s,1H),8.34(d,J=5.5Hz,1H), 7.41(d,J=8.1Hz,1H),7.49-7.31(m,5H),7.29(s, 1H), 7.23(d,J=7.3Hz,1H),6.77(dd,J=1.2Hz,5.5Hz,1H),4.09(s,2H), 2.39(s,3H)
Mass,m/e:448($M^+$),236(base)

Preparation Example 10

3-(3-Methylphenyl)-5-[(2-methylphenyl)acetylamino]-4-(4-pyrimidinyl)isoxazole (Compound No. B-9)

$^1$H-NMR($CDCl_3$)δ:11.38(s,1H),8.40(d,J=1.4Hz,1H), 8.31(d,J=5.6Hz,1H),7.41-7.31(m,6H),7.27(bs,1H), 7.21 (dd,J=7.7Hz,1H),6.74(dd,J=1.4Hz,5.6Hz,1H), 3.92(s,2H),2.38(s,3H),2.36(s,3H)
Mass,m/e:384($M^+$),236(base)

Preparation Example 11

3-(3-Methylphenyl)-5-[(3-methylphenyl)acetylamino]-4-(4-pyrimidinyl)isoxazole (Compound No. B-10)

$^1$H-NMR($CDCl_3$)δ:11.49(s,1H),8.47(s,1H),8.33(d,J=5.4Hz,1H), 7.39-7.32(m,3H),7.28-7.19(m,5H),6.76(dd, J=1.5Hz,5.4Hz,1H),3 .89(s,2H),2.39(s,6H)
Mass,m/e:384($M^+$),236(base)

Preparation Example 12

3-(2-Fluoro-5-methylphenyl)-5-(phenylacetylamino)-4-(4-pyrimidinyl)isoxazole (Compound B-11)

$^1$H-NMR($CDCl_3$)δ:11.46(bs,1H),8.47(s,1H),8.37(d,J=5.4Hz,1H), 7.51-7.40(m,5H),7.34-7.28(m,2H),7.07(t, J=9.3Hz,1H), 6.70(td,J= 1.9Hz, 5.4Hz, 1H),3.94(s,2H),2.37(s,3H)
Mass,m/e:388($M^+$),254(base)

Preparation Example 13

5-[(3-Methoxyphenyl)acetylamino]-3-(3-methyl-4-fluorophenyl)-4-(4-pyrimidinyl)isoxazole (Compound No. B-12)

$^1$H-NMR($CDCl_3$)δ:11.41(s,1H),8.59(s,1H),8.36(d,J=5.8Hz,1H), 7.39(t,J=8.1Hz, 1H), 7.31(d,J=7.0Hz, 1H), 7.25-7.22(m, 1H), 7.11(t,J=9.3Hz,1H),6.98-6.94(m,3H), 6.76(dd,J=1.2Hz,5.8Hz,1H),3.89(s,2H),3.83(s,3H),2.31(s,3H)
Mass,m/e:418($M^+$),254(base)

Preparation Example 14

3-(3-Methyl-4-fluorophenyl)-5-[(2-methylphenyl)acetylamino]-4-(4-pyrimidinyl)isoxazole (Compound No. B-13)

$^1$H-NMR($CDCl_3$)δ:11.35(bs,1H),8.41(d,J=1.4Hz,1H), 8.34(d,J=5.4Hz,1H),7.41-7.30(m,5H),7.24-7.20(m,1H), 7.11(t,J=9.3Hz,1H),6.74(dd,J=1.4Hz,5.4Hz,1H),3.92(s,2H), 2.35(s,3H),2.30(d,J=1.9Hz,3H)
Mass,m/e:402($M^+$),254(base)

Preparation Example 15

3-(3-Methylphen-yl)-5-(3-phenylpropionylamino)-4-(4-pyridyl)isoxazole (Compound No. B-14)

Preparation Example 1-c was repeated except that 4-pyrimidinylacetonitrile was replaced by 4-pyridylacetonitrile, to synthesize 5-amino-3-(3-methylphenyl)-4-(4-pyridyl)isoxazole, from which then the title compound was synthesized in the manner similar to Preparation Example 1-d.
$^1$H-NMR($CDCl_3$)δ:8.39(dd,J=1.5Hz,4.6Hz,2H),8.34(bs,1H), 7.30-7.16(m,8H),7.03(d,J=7.3Hz,1H), 6.94(dd,J=1.5Hz, 4.6Hz,2H),3.00(t,J=7.3Hz,2H), 2.75(t,J=7.3Hz,2H),2.29(s,3H)
Mass,m/e:383($M^+$),91(base)
Hereinafter the compounds of Preparation Examples 16 - 20 were synthesized in the manner similar to Preparation Example 15.

Preparation Example 16

3-(3-Methylphenyl)-5-[(2-methylphenyl)propionylamino]-4-(4-pyridyl)isoxazole (Compound B-15)

$^1$H-NMR($CDCl_3$)δ:8.44(d,J=6.0Hz,2H),8.03(bs,1H), 7.24-7.18(m,3H),7.15-7.10(m,4H),7.05(d,J=7.03Hz,1H), 6.97(d,J=6.0Hz,2H),2.99(t,J=7.6Hz,2H),2.71(t,J=7.6Hz,2H), 2.30(s,3H),2.29(s,3H)
Mass,m/e:397($M^+$),105(base)

Preparation Example 17

5-[(3-Chlorophenyl)propionylamino]-3-(2-fluoro-5-methylphenyl)-4-(4-pyridyl)isoxazole (Compound No. B-16)

$^1$H-NMR($CDCl_3$)δ:8.41(d,J=5.8Hz,2H),8.07(bs,1H), 7.28(dd,J=1.7Hz,6.2Hz,1H),7.23-7.19(m,4H),7.08-7.06 (m,1H), 6.92-6.88(m,3H),2.99(t,J=7.4Hz,2H),2.76(t,J=7.4Hz,2H), 2.33(s,3H)
Mass,m/e:435($M^+$),269(base)

Preparation Example 18

3-(4-Fluoro-3-methylphenyl)-5-(phenylacetylamino)-4-(4-pyridyl)isoxazole (Compound No. B-17)

$^1$H-NMR($CDCl_3$)δ:8.45(dd,J=1.5Hz,4.6Hz,2H),7.60(bs,1H), 7.42-7.33(m,3H),7.29-7.22(m,3H),7.08-7.03(m, 1H), 6.94(t,J=9.1Hz,1H),6.89(dd,J=1.5Hz,4.6Hz,2H),3.76(s,2H), 2.22(d,J=1.9Hz,3H)
Mass,m/e:387($M^+$),91(base)

Preparation Example 19

5-[(2-Chlorophenyl)acetylaminol-3-(4-fluoro-3-methylphenyl)-4-(4-pyridyl)isoxazole (Compound No. B-18)

$^1$H-NMR($CDCl_3$)δ:8.49(dd,J=1.7Hz,4.4Hz,2H),7.63(bs,1H), 7.45-7.42(m,1H),7.35-7.26(m,4H),7.09-7.05(m, 1H), 6.98(dd,J=1.7Hz,4.4Hz,2H),6.95(t,J=8.9Hz,1H),3.87(s,2H), 2.22(d,J=1.9Hz,3H)
Mass,m/e:421($M^+$),125(base)

Preparation Example 20

3-(4-Fluoro-3-methlyphenyl)-5-(3-phenylpropionylamino)-4-(4-pyridyl)isoxazole (Compound No. B-19)

$^1$H-NMR($CDCl_3$)δ:8.49(d,J=5.8Hz,2H),7.69(s,1H), 7.32-7.16(m,5H),7.09-7.04(m,1H),6.98-6.94(m,3H), 3.01(t, J=7.4Hz,2H),2.77(t,J=7.4Hz,2H),2.23(s,3H)
Mass,m/e:401($M^+$),91(base)

## Claims

1. Treating agents of inflammatory bowel disease, which are **characterized by** containing p38MAPkinase inhibitor having properties of antedrug as the active ingredient.

**2.** Treating agents according to Claim 1, in which the p38MAPkinase inhibitor having properties of antedrug is selected from:

compounds represented by a formula (I)

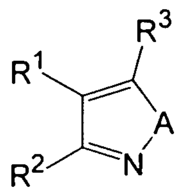

( I )

in the formula,

$R^1$ stands for a substituted or unsubstituted 6-membered aromatic heterocyclic group,
$R^2$ stands for a substituted or unsubstituted aromatic carbocyclic group or aromatic heterocyclic group,
$R^3$ stands for a substituted or unsubstituted carbocyclic group or heterocyclic group, having a straight chain linker, the chain being constituted of 1 - 5 atoms selected from carbon, oxygen and nitrogen, and
A stands for carbon atom, oxygen atom, sulfur atom or nitrogen atom, or salts thereof;

compounds represented by a formula (II)

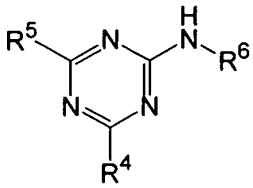

( II )

in the formula,

$R^4$ stands for a heterocyclic group linked directly or through a linker to triazine ring,
$R^5$ stands for a substituted or unsubstituted amino group, and
$R^6$ stands for a substituted or unsubstituted phenyl group, or salts thereof; and

compounds represented by a formula (III)

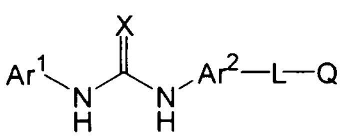

( III )

in the formula,

$Ar^1$ stands for a substituted or unsubstituted aromatic carbocyclic group or aromatic heterocyclic group,
$Ar^2$ stands for a substituted or unsubstituted aromatic carbocycle or heterocycle,
X stands for O or S,
L stands for a linker whose chain is constituted of 1 - 3 atoms selected from carbon, oxygen and sulfur, and
Q stands for a substituted or unsubstituted heterocyclic group,

or salts thereof.

**3.** Treating agents according to Claim 1 containing the p38MAPkinase inhibitor having properties of antedrug selected from

5-[(2-chlorophenyl)acetylamino]-3-(4-fluorophenyl)-4-(4-pyrimidinyl)isoxazole,
5-[(2-chloro-6-fluorophenyl)acetylamino]-3-(4-fluorophenyl)-4-(4-pyrimidinyl)isoxazole,
3-(4-chlorophenyl)-5-[(2-chlorophenyl)acetylamino]-4-(4-pyrimidinyl)isoxazole,
5-[(2-chlorophenyl)acetylamino]-3-(2,4-difluorophenyl)-4-(4-pyrimidinyl)isoxazole,
3-(2,4-difluorophenyl)-5-[(3-methylphenyl)acetylamino]-4-(4-pyrimidinyl)isoxazole,
5-[(2-chlorophenyl)acetylamino]-3-(2-fluoro-4-methoxyphenyl)-4-(4-pyrimidinyl)isoxazole,
5-[(2-chlorophenyl)acetylamino]-3-(2,3-methylenedioxyphenyl)-4-(4-pyrimidinyl)isoxazole,
5-[(2-chlorophenyl)acetylamino]-3-(3-methylphenyl)-4-(4-pyrimidinyl)isoxazole,
5-[(2-bromophenyl)acetylamino]-3-(3-methylphenyl)-4-(4-pyrimidinyl)isoxazole,
3-(3-methylphenyl)-5-[(2-methylphenyl)acetylamino]-4-(4-pyrimidinyl)isoxazole,
3-(3-methylphenyl)-5-[(3-methylphenyl)acetylamino]-4-(4-pyrimidinyl)isoxazole,
3-(2-fluoro-5-methylphenyl)-5-(phenylacetylamino)-4-(4-pyrimidinyl)isoxazole,
5-[(3-methoxyphenyl)acetylamino]-3-(3-methyl-4-fluorophenyl)-4-(4-pyrimidinyl)isoxazole,
3-(3-methyl-4-fluorophenyl)-5-[(2-methylphenyl)acetylamino]-4-(4-pyrimidinyl)isoxazole,
3-(3-methylphenyl)-5-(3-phenylpropionylamino)-4-(4-pyridyl)isoxazole,
3-(3-methylphenyl)-5-[(2-methylphenyl)propionylamino]-4-(4-pyridyl)isoxazole,
5-[(3-chlorophenyl)propionylamino]-3-(2-fluoro-5-methylphenyl)-4-(4-pyridyl)isoxazole,
3-(4-fluoro-3-methylphenyl)-5-phenylacetylamino-4-(4-pyridyl)isoxazole,
5-[(2-chlorophenyl)acetylamino]-3-(4-fluoro-3-methylphenyl)-4-(4-pyridyl)isoxazole,
3-(4-fluoro-3-methylphenyl)-5-(3-phenylpropionylamino)-4-(4-pyridyl)isoxazole,
4-(4-fluorophenyl)-2-(4-hydroxy-3,5-diphenyl)-5-(4-pyridyl)-imidazole,
4-(4-fluorophenyl)-2-(4-methanesulfonylphenyl)-5-(4-pyridyl)-imidazole,
3-(4-fluorophenyl)-5-phenylacetylamino-4-(4-pyridyl)pyrazole,
3-(4-fluorophenyl)-5-(4-methoxyphenylacetylamino)-4-(4-pyridyl)pyrazole,
5-(2-chlorophenylacetylamino)-3-(4-fluorophenyl)-4-(4-pyridyl)pyrazole,
5-(2,5-difluorophenylacetylamino)-3-(4-fluorophenyl)-1-methyl-4-(4-pyridyl)pyrazole,
5-(2-chloro-6-fluorophenylacetylamino)-3-(4-fluorophenyl)-1-methyl-4-(4-pyridyl)pyrazole,
5-(2,6-dichlorophenylacetylamino)-3-(4-fluorophenyl)-1-methyl-4-(4-pyridyl)pyrazole,
5-(2,4-dichlorophenylacetylamino)-3-(4-fluorophenyl)-1-methyl-4-(4-pyridyl)pyrazole,
5-(2,6-difluorophenylacetylamino)-3-(4-fluorophenyl)-1-methyl-4-(4-pyridyl)pyrazole,
5-(3,5-difluorophenylacetylamino)-3-(4-fluorophenyl)-1-methyl-4-(4-pyridyl)pyrazole,
5-(2,3-difluorophenylacetylamino)-3-(4-fluorophenyl)-1-methyl-4-(4-pyridyl)pyrazole,
5-(2,5-dimethylphenylacetylamino)-3-(4-fluorophenyl)-1-methyl-4-(4-pyridyl)pyrazole,
5-(2,4-dimethoxyphenylacetylamino)-3-(4-fluorophenyl)-1-methyl-4-(4-pyridyl)pyrazole,
5-(2-chlorophenylacetylamino)-3-(4-fluorophenyl)-1-methyl-4-(4-pyridyl)pyrazole,
5-(2-bromophenylacetylamino)-3-(4-fluorophenyl)-1-methyl-4-(4-pyridyl)pyrazole,
3-(4-fluorophenyl)-5-(2-fluorophenylacetylamino)-1-methyl-4-(4-pyridyl)pyrazole,
3-(4-fluorophenyl)-1-methyl-5-(2-methylphenylacetylamino)-4-(4-pyridyl)pyrazole,
3-(4-fluorophenyl)-5-(2-methoxyphenylacetylamino)-1-methyl-4-(4-pyridyl)pyrazole,
3-(4-fluorophenyl)-5-(2-nitrophenylacetylamino)-4-(4-pyridyl)-pyrazole,
1-ethyl-3-(4-fluorophenyl)-5-phenylacetylamino-4-(4-pyridyl)-pyrazole,
3-(4-fluorophenyl)-5-phenylacetylamino-1-propyl-4-(4-pyridyl)pyrazole,
3-(4-fluorophenyl)-1-(2-hydroxyethyl)-5-(2-methoxyphenyl-acetylamino)-4-(4-pyridyl)pyrazole,
5-(2-chlorophenylacetylamino)-3-(4-fluorophenyl)-1-(2-hydroxyethyl)-4-(4-pyridyl)pyrazole,
1-methyl-3-(3,4-methylenedioxyphenyl)-5-phenylacetylamino-4-(4-pyridyl)pyrazole,
3-(4-fluorophenyl)-1-methyl-5-phenylacetylamino-4-(4-pyridyl)pyrazole,
3-(4-fluorophenyl)-1-methyl-5-(N-methyl-N-phenylacetyl-amino)-4-(4-pyridyl)pyrazole,
3-(4-fluorophenyl)-5-(N-formyl-N-phenethylamino)-1-methyl-4-(4-pyridyl)pyrazole,
3-(4-fluorophenyl)-5-(1-hydroxy-4-phenylbutyl)-4-(4-pyridyl)-pyrazole,
3-(4-fluorophenyl)-5-[1-hydroxy-3-(3-tolyl)propyl]-4-(4-pyridyl)pyrazole,
3-(4-fluorophenyl)-5-(1-hydroxy-3-phenylbutyl)-4-(4-pyridyl)-pyrazole,
3-(4-fluorophenyl)-5-(1-hydroxy-2-methyl-3-phenylpropyl)-4-(4-pyridyl)pyrazole,
3-(4-fluorophenyl)-5-(1-hydroxy-3-phenylpropyl)-1-methyl-4-(4-pyridyl)pyrazole,
3-(4-fluorophenyl)-4-(4-pyridyl)-5-[3-(2-pyridyl)-1-propenyl]-pyrazole,
3-(4-fluorophenyl)-4-(4-pyridyl)-5-[3-(3-pyridyl)-1-propenyl]-pyrazole,

3-(4-fluorophenyl)-5-[2-methyl-3-(3-pyridyl)-1-propenyl]-4-(4-pyridyl)pyrazole,
3-(4-fluorophenyl)-4-(4-pyridyl)-5-[3-(2-pyridyl)-1-butenyl]-pyrazole,
3-(4-fluorophenyl)-1-methyl-4-(4-pyridyl)-5-[3-(3-pyridyl)-1-propenyl]pyrazole,
3-(4-fluorophenyl)-5-(3-phenylpropyl)-4-(4-pyridyl)pyrazole,
3-(4-fluorophenyl)-4-(4-pyridyl)-5-[3-(3-pyridyl)butyl]pyrazole,
3-(4-fluorophenyl)-4-(4-pyridyl)-5-[3-(2-pyridyl)propyl]-pyrazole,
3-(4-fluorophenyl)-5- [2-methyl-3-(3-pyridyl)propyl]-4-(4-pyridyl)pyrazole,
3-(4-fluorophenyl)-4-(4-pyridyl)-5-[3-(2-pyridyl)butyl]pyrazole,
3-(4-fluorophenyl)-4-(4-pyridyl)-5-[3-(3-pyridyl)propyl]-pyrazole,
5-(3-phenylpropyl)-3-(2-pyridyl)-4-(4-pyridyl)pyrazole,
3-(4-fluorophenyl)-5-[3-(4-nitrophenyl)propyl]-4-(4-pyridyl)-pyrazole,
5-[3-(4-aminophenyl)propyl]3-(4-fluorophenyl)-4-(4-pyridyl)-pyrazole,
3-(4-fluorophenyl)-5-[3-(1-pyrazolyl)propyl]-4-(4-pyridyl)-pyrazole,
5-[3-(4-aminophenyl)propyl]-3-(4-fluorophenyl)-1-methyl-4-(4-pyridyl)pyrazole,
3-(4-fluorophenyl)-1-methyl-5-(3-phenylpropyl)-4-(4-pyridyl)-pyrazole,
3-(4-fluorophenyl)-1-(2-hydroxyethyl)-5-(3-phenylpropyl)-4-(4-pyridyl)pyrazole,
3-(4-fluorophenyl)-1-(2-dimethylaminoethyl)-5-(3-phenylpropyl)-4-(4-pyridyl)pyrazole,
3-(4-fluorophenyl)-5-(N-methyl-4-methoxybenzylamino-carbonyl)-4-(4-pyridyl)pyrazole,
5-(N-ethylbenzylaminocarbonyl)-3-(4-fluorophenyl)-4-(4-pyridyl)pyrazole,
1-ethyl-5-(N-methylbenzylaminocarbonyl)-3-(4-fluorophenyl)-4-(4-pyridyl)pyrazole,
1-ethyl-5-(N-ethylbenzylaminocarbonyl)-3-(4-fluorophenyl)-4-(4-pyridyl)pyrazole,
3-(4-fluorophenyl)-1-methyl-5-(N-methylbenzylaminocarbonyl)-4-(4-pyridyl)pyrazole,
5-(benzyloxymethyl)-3-(4-fluorophenyl)-4-(4-pyridyl)pyrazole,
5-[1-(benzyloxy)ethyl]-3-(4-fluorophenyl)-4-(4-pyridyl)pyrazole,
3-(4-fluorophenyl)-5-(2-oxo-3-phenylpropyl)-4-(4-pyridyl)-pyrazole,
3-(4-fluoropheyl)-5-(3-hydroxy-3-phenylpropyl)-4-(4-pyridyl)-pyrazole,
4-methyl-3-[4-[N-methyl-N-(2-phenylpropyl)amino]-6-((S)-pyrrolidin-3-ylamino)-1,3,5-triazin-2-ylamino]benzamide,
4-methyl-3-[4-isopropylamino-6-((S)-pyrrolidin-3-ylamino)-1,3,5-triazin-2-ylamino]benzamide,
4-methyl-3-[4-(N-methyl-N-isopropylamino)-6-((S)-pyrrolidin-3-ylamino)-1,3,5-triazin-2-ylamino]benzamide,
4-methyl-3-[4-(N-methyl-N-neopentylamino)-6-((S)-pyrrolidin-3-ylamino)-1,3,5-triazin-2-ylamino]benzamide,
N-methoxy-4-methyl-3-[4-(N-methyl-N-neopentylamino)-6-(4-methylpiperazin-1-yl)-1,3,5-triazin-2-ylamino]benzamide,
N-methoxy-4-methyl-3-[4-methyl-N-neopentylamino]-6-(piperazin-1-yl)-1,3,5-triazin-2-ylaminolbenzamide,
N-methoxy-4-methyl-3-[4-(N-methyl-N-neopentylamino)-6-(4-methyl-1,4-diazepan-1-yl)-1,3,5-triazin-2-ylamino]benzamide,
N-methoxy-4-methyl-3-[4-(N-methyl-N-neopentylamino)-6-(1,4-diazepan-1-yl)-1,3,5-triazin-2-ylamino]benzamide,
N-ethyl-4-methyl-3-[4-(N-methyl-N-neopentylamino)-6-((S)-pyrrolidin-3-ylamino)-1,3,5-triazin-2-ylamino]benzamide,
N-benzyl-4-methyl-3-[4-(N-methyl-N-neopentylamino)-6-((S)-pyrrolidin-3-ylamino)-1,3,5-triazin-2-ylamino]benzamide,
N-methoxy-4-methyl-3-[4-(N-methyl-N-neopentylamino)-6-((S)-pyrrolidin-3-ylamino)-1,3,5-triazin-2-ylamino]benzamide,
N-hydroxy-4-methyl-3-[4-(N-methyl-N-neopentylamino)-6-((S)-pyrrolidin-3-ylamino)-1,3,5-triazin-2-ylamino]benzamide,
N-isopropyl-4-methyl-3-[4-(N-methyl-N-neopentylamino)-6-((S)-pyrrolidin-3-ylamino)-1,3,5-triazin-2-ylamino]benzamide,
[4-(2-cyanopyridin-4-ylmethyl)-2-fluorophenyl]amino-N-(2-naphthyl)carboxamide,
[3-chloro-4-(2-cyanopyridin-4-ylmethyl)phenyl]amino-N-(2,2-difluorobenzo[d]-1,3-dioxan-5-yl)carboxamide,
[3-chloro-4-(2-cyanopyridin-4-yloxy)phenyl]amino-N-(2,2-difluorobenzo[d]-1,3-dioxan-5-yl)carboxamide,
[3-chloro-4-(2-cyanopyridin-4-ylthio)phenyl]amino-N-(2,2-difluorobenzo[d]1,3-dioxan-5.yl)carboxamide,
[4-(2-cyanopyridin-4-ylmethyl)-3-methylphenyl]amino-N-(2,2,3,3-tetrafluorobenzo[e]-1,4-dioxan-6-yl)carboxamide,
[4-(2-cyanopyridin-4-yloxy)-3-methylphenyl]amino-N-(2,2,3,3-tetrafluorobenzo[e]-1,4-dioxan-6-yl)carboxamide,
[4-(2-cyanopyridin-4-yloxy)-2-fluorophenyl]amino-N-(2-trifluoromethylpyridin-4-yl)carboxamide,
[4-(2-cyanopyridin-4-yloxy)phenyl]amino-N-(4-tert-butylpyridin-2-yl)carboxamide,

[4-(2-cyanopyridin-4-yloxy)-3-fluorophenyl]amino-N-(4-tert-butylpyridin-2-yl)carboxamide,
[4-(2-cyanopyridin-4-yloxy)phenyl]amino-N-(4-ethylpyridin-2-yl)carboxamide,
[2-chloro-4-(2-cyanopyridin-4-yloxy)phenyl]amino-N-quinolin-6-ylcarboxamide,
[3-chloro-4-(2-cyanopyridin-4-yloxy)phenyl]amino-N-isoquinolin-3-ylcarboxamide,
[4-(2-cyanopyridin-4-yloxy)-2-nitrophenyl]amino-N-benzothiazol-5-ylcarboxamide, and
[4-(3-methylaminocarbonylpyridin-4-yloxy)-2-fluorophenyl]-amino-N-(3-trifluoromethyl-4-chlorophenyl)carboxamide.

**4.** Pharmaceutical compositions for treating inflammatory bowel disease, which contain p38MAPkinase inhibitor having properties of antedrug concurrently with non-toxic excipients.

**5.** A treating method of inflammatory bowel disease, which is **characterized by** administering an effective dose of p38MAPkinase inhibitor having properties of antedrug to a patient who needs the treatment.

**INTERNATIONAL SEARCH REPORT**

International application No.
PCT/JP2007/063206

A. CLASSIFICATION OF SUBJECT MATTER
*A61K45/00*(2006.01)i, *C07D401/04*(2006.01)i, *A61K31/4439*(2006.01)i, *A61K31/444*(2006.01)i, *A61K31/506*(2006.01)i, *A61P1/00*(2006.01)i, *A61P1/04*(2006.01)i, *A61P11/00*(2006.01)i, *A61P11/02*(2006.01)i,

According to International Patent Classification (IPC) or to both national classification and IPC

B. FIELDS SEARCHED

Minimum documentation searched (classification system followed by classification symbols)
C07D401/04, A61K31/4439, A61K31/444, A61K31/506, A61K45/00, A61P1/00, A61P1/04, A61P11/00, A61P11/02, A61P11/06, A61P17/00, A61P19/02, A61P29/00, A61P37/08, A61P43/00, C07D401/14, C07D413/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAOLD(STN), CAplus(STN), REGISTRY(STN), MEDLINE(DIALOG), PubMed, Science Direct, JCHEM(JDream2), JMEDPlus(JDream2), JST7580(JDream2),

C. DOCUMENTS CONSIDERED TO BE RELEVANT

| Category* | Citation of document, with indication, where appropriate, of the relevant passages | Relevant to claim No. |
|---|---|---|
| X | JP 2000-086657 A (TEIKOKU HORMONE MFG CO., LTD.),<br>28 March, 2000 (28.03.00),<br>Page 23; example 61<br>(Family: none) | 1-4 |
| X | WO 2000/75131 A1 (TEIKOKU HORMONE MFG CO., LTD.),<br>14 December, 2000 (14.12.00),<br>Page 32; example 3<br>& EP 1188754 A1 & JP 2001-502414 A<br>& US 6667325 B1 & US 2004/087628 A1<br>& EP 1188754 B1 & US 7087624 B2 | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>20 July, 2007 (20.07.07) | Date of mailing of the international search report<br>31 July, 2007 (31.07.07) |
| Name and mailing address of the ISA/<br>Japanese Patent Office<br><br>Facsimile No. | Authorized officer<br><br><br>Telephone No. |

Form PCT/ISA/210 (second sheet) (April 2005)

**INTERNATIONAL SEARCH REPORT**

International application No.
PCT/JP2007/063206

C (Continuation). DOCUMENTS CONSIDERED TO BE RELEVANT

| Category* | Citation of document, with indication, where appropriate, of the relevant passages | Relevant to claim No. |
|---|---|---|
| X | WO 2000/39116 A1 (TEIKOKU HORMONE MFG CO., LTD.), 06 July, 2000 (06.07.00), Page 31; example 3 & EP 1142890 A1 & JP 2000-591027 A & US 6511997 B1 & EP 1142890 B1 | 1-4 |
| X | JP 2002-508754 A (G.D. SEARLE AND CO.), 19 March, 2002 (19.03.02), Page 424; example B-0001 & WO 98/52940 A1 & EP 1000055 A1 & US 6423713 B1 & US 6617324 B1 & US 2004/176433 A1 & US 7071198 B1 & US 7153959 B2 | 1-4 |
| X | JP 2002-530397 A (G.D. SEARLE AND CO.), 17 September, 2002 (17.09.02), Page 1140; example C-117 & WO 2000/31063 A1 & EP 1144403 A1 & US 6514977 B1 & US 6525059 B1 & EP 1144403 B1 & EP 1500657 A1 | 1-4 |
| X | LEFTHERIS,K. et al, The discovery of orally active triaminotriazine aniline amides as inhibitors of p38 MAP kinase, J Med Chem, 2004, Vol.47, No.25, p.6283-6291 | 1-4 |
| X | REGAN,J. et al, Pyrazole urea-based inhibitors of p38 MAP kinase: from lead compound to clinical candidate, J Med Chem, 2002, Vol.45, No.14, p.2994-3008 | 1-4 |
| A | Tetsuya AOKI, "Steroid antedrug - Nanjisei Kaiyosei Daichoen ni Okeru Chiryoho toshite", Igaku no Ayumi, 1996, Vol.178, No.9, pages 700 to 701 | 1-4 |
| P,X | WO 2006/070927 A1 (ASKA PHARM CO., LTD., JP), 06 July, 2006 (06.07.06), (Family: none) | 1-4 |

Form PCT/ISA/210 (continuation of second sheet) (April 2005)

**INTERNATIONAL SEARCH REPORT**

International application No.
PCT/JP2007/063206

Continuation of A. CLASSIFICATION OF SUBJECT MATTER
(International Patent Classification (IPC))

*A61P11/06*(2006.01)i, *A61P17/00*(2006.01)i, *A61P19/02*(2006.01)i, *A61P29/00*(2006.01)i, *A61P37/08*(2006.01)i, *A61P43/00*(2006.01)i, *C07D401/14*(2006.01)i, *C07D413/04*(2006.01)i

(According to International Patent Classification (IPC) or to both national classification and IPC)

Continuation of B. FIELDS SEARCHED
Electronic data base consulted during the international search (name of data base and, where practicable, search terms used)

JSTPlus(JDream2)

Form PCT/ISA/210 (extra sheet) (April 2005)

**INTERNATIONAL SEARCH REPORT**

International application No.
PCT/JP2007/063206

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 5
because they relate to subject matter not required to be searched by this Authority, namely:
Claim 5 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
The matter common to claims 1-4, "a treatment agent for inflammatory bowel disease containing, as an active ingredient, a p38 MAP kinase inhibitor having a property of an antedrug" is known as described also in the document shown in the Box C, therefore, this matter is not a technical feature which makes a contribution over the prior art. Further, there is no other common matter considered to be a special technical feature in the inventions of claims 1-4.
Accordingly, claims 1-4 do not share a special technical feature, therefore, these group of inventions are not so linked as to form a single general inventive concept.

1. ☐ As all required additional search fees were timely paid by the applicant, this international search report covers all searchable claims.
2. ☒ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the
☐ The additional search fees were accompanied by the applicant's protest and, where applicable, payment of a protest fee..
☐ The additional search fees were accompanied by the applicant's protest but the applicable protest fee was not paid within the time limit specified in the invitation.

Form PCT/ISA/210 (continuation of first sheet (2)) (April 2005)

**INTERNATIONAL SEARCH REPORT**

International application No.
PCT/JP2007/063206

<Regarding the subject of search>

Claims 1-4 relate to a treatment agent for inflammatory bowel disease containing, as an active ingredient, a compound defined by a desired property of "a p38 MAP kinase inhibitor having a property of an antedrug".

Further, claims 1 and 4 include every compound having such a property, and claims 2 and 3 describe also a structural formula and a chemical formula, respectively. However, what is supported by the description within the meaning of PCT Article 6 and disclosed within the meaning of PCT Article 5 among the compounds having the above property is only a specified small part, a part of isoxazole derivatives and pyrazole derivatives.

Further, with regard to the "p38 MAP kinase inhibitor having a property of an antedrug", the scope of the compound having such a property cannot be specified even if the technical knowledge at the time of filing is considered. Therefore, claims 1-4 also lack the requirement of clarity under PCT Article 6.

Thus, a search was carried out for the relationship between the p38 MAP kinase inhibitor and inflammatory bowel disease, and a part related to the treatment agent for inflammatory bowel disease containing, as an active ingredient an isoxazole derivative or a pyrazole derivative described specifically in the description.

Incidentally, as a result of the search, the invention is known as described in the document shown in the Box C, and includes a lot of known compounds, therefore, for the presentation of reference documents, those describing compounds specifically described in the invention are mainly shown.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9314081 A
- WO 9852940 A
- WO 0039116 A
- JP 2000086657 A
- WO 9625405 A
- WO 200417968 A
- WO 200422555 A
- WO 0064894 A
- WO 200472072 A
- WO 200414907 A
- WO 200473628 A
- WO 200573189 A
- WO 200585249 A
- WO 200400846 A
- WO 0075131 A
- WO 2004078747 A


### Non-patent literature cited in the description


- *Bioorganic & Medicinal Chemistery,* 1997, vol. 5 (1), 49-64
- *J. Med. Chem.,* 2004, vol. 47, 6283-6291